(19) Europäisches Patentamt European Patent Office Office européen des brevets

(11) **EP 1 951 899 B1**

(12) **EUROPEAN PATENT SPECIFICATION**

(45) Date of publication and mention of the grant of the patent:
**30.06.2010 Bulletin 2010/26**

(21) Application number: **06809745.0**

(22) Date of filing: **27.11.2006**

(51) Int Cl.:
*C12Q 1/68* (2006.01)

(86) International application number:
**PCT/IE2006/000133**

(87) International publication number:
**WO 2007/060647 (31.05.2007 Gazette 2007/22)**

(54) **A METHOD FOR DETECTING OR MONITORING SEPSIS BY ANALYSING CYTOKINE MRNA EXPRESSION LEVELS**

VERFAHREN ZUR DIAGNOSE VON SEPSIS DURCH ANALYSE VON CYTOKINE MRNA EXPRESSION

METHODE DE DIAGNOSTIC DE SEPSIE PAR ANALYSE D'EXPRESSION DE MRNA DES CYTOKINES

(84) Designated Contracting States:
**AT BE BG CH CY CZ DE DK EE ES FI FR GB GR HU IE IS IT LI LT LU LV MC NL PL PT RO SE SI SK TR**

(30) Priority: **25.11.2005 IE 20050783**

(43) Date of publication of application:
**06.08.2008 Bulletin 2008/32**

(73) Proprietor: **The Provost, Fellows and Scholars of the College of the Holy and Undivided Trinity of Queen Elizabeth near Dublin Dublin 2 (IE)**

(72) Inventors:
• **RYAN, Thomas**
  **Dublin 15 (IE)**
• **KELLEHER, Dermot**
  **Dun Laoghaire**
  **County Dublin (IE)**
• **MCMANUS, Owen, Ross**
  **Killiney**
  **County Dublin (IE)**
• **O'DWYER, Michael**
  **Dublin 3 (IE)**
• **STORDEUR, Patrick**
  **B-6010 Charleroi (BE)**

(74) Representative: **Purdy, Hugh Barry et al**
  **PurdyLucey**
  **Intellectual Property**
  **The Capel Building, Suite 138-139**
  **Mary's Abbey**
  **Dublin 7 (IE)**

(56) References cited:
**EP-A2- 1 310 567**     **WO-A-97/48728**
**WO-A-2006/061644**     **WO-A2-2004/044556**
**WO-A2-2004/108957**

• **BERNER REINHARD ET AL: "Elevated gene expression of interleukin-8 in cord blood is a sensitive marker for neonatal infection" EUROPEAN JOURNAL OF PEDIATRICS, vol. 159, no. 3, March 2000 (2000-03), pages 205-210, XP002419700 ISSN: 0340-6199**
• **PACHOT A ET AL: "Longitudinal study of cytokine and immune transcription factor mRNA expression in septic shock" CLINICAL IMMUNOLOGY, ACADEMIC PRESS,, US, vol. 114, no. 1, January 2005 (2005-01), pages 61-69, XP004679966 ISSN: 1521-6616**
• **OBERHOFFER M ET AL: "Procalcitonin expression in human peripheral blood mononuclear cells and its modulation by lipopolysaccharides and sepsis-related cytokines in vitro." THE JOURNAL OF LABORATORY AND CLINICAL MEDICINE JUL 1999, vol. 134, no. 1, July 1999 (1999-07), pages 49-55, XP002419701 ISSN: 0022-2143**

EP 1 951 899 B1

- KOX W J ET AL: "Immunomodulatory therapies in sepsis." INTENSIVE CARE MEDICINE 2000, vol. 26 Suppl 1, 2000, pages S124-S128, XP002419703 ISSN: 0342-4642
- STEINHAUSER M L ET AL: "IL-10 is a major mediator of sepsis-induced impairment in lung antibacterial host defense." JOURNAL OF IMMUNOLOGY (BALTIMORE, MD. : 1950) 1 JAN 1999, vol. 162, no. 1, 1 January 1999 (1999-01-01), pages 392-399, XP002419732 ISSN: 0022-1767
- WIRTZ STEFAN J ET AL: "A key pathogenic role of Il-27/wsx-1 signalling in experimental septic peritonitis" GASTROENTEROLOGY, vol. 128, no. 4, Suppl. 2, April 2005 (2005-04), page A69, XP002432848 & ANNUAL MEETING OF THE AMERICAN-GASTROENTEROLOGICAL-ASSOCIATION/D IGEST IVE-DISEASE-WEEK; CHICAGO, IL, USA; MAY 14 -19, 2005 ISSN: 0016-5085
- CUA D J ET AL: "Interleukin-23 rather than interleukin-12 is the critical cytokine for autoimmune inflammation of the brain" NATURE, NATURE PUBLISHING GROUP, LONDON, GB, vol. 421, 13 February 2003 (2003-02-13), pages 744-748, XP002971969 ISSN: 0028-0836
- O'DWYER MICHAEL J ET AL: "The occurrence of severe sepsis and septic shock are related to distinct patterns of cytokine gene expression." SHOCK (AUGUSTA, GA.) DEC 2006, vol. 26, no. 6, December 2006 (2006-12), pages 544-550, XP009078725 ISSN: 1073-2322
- PACHOT ET AL: "Systemic transcriptional analysis in survivor and non-survivor septic shock patients: A preliminary study" IMMUNOLOGY LETTERS, AMSTERDAM, NL, vol. 106, no. 1, 15 July 2006 (2006-07-15), pages 63-71, XP005572590 ISSN: 0165-2478 cited in the application
- "Abstracts of the AGA Institute" GASTROENTEROLOGY, ELSEVIER, PHILADELPHIA, PA, US, vol. 130, no. 4, April 2006 (2006-04), pages A1-A747, XP005643844 ISSN: 0016-5085 & WIRTZ STEFAN J ET AL: "Protection from lethal septic peritonitis by neutralizing the biological function of Interleukin-27" GASTROENTEROLOGY, vol. 130, no. 4, Suppl. 2, April 2006 (2006-04), page A49, & DIGESTIVE DISEASE WEEK MEETING/107TH ANNUAL MEETING OF THE AMERICAN-GASTROENTEROLOGICAL-ASSOCIATION; LOS ANGELES, CA, USA; MAY 19 24, 2006 ISSN: 0016-5085
- WIRTZ STEFAN ET AL: "Protection from lethal septic peritonitis by neutralizing the biological function of interleukin 27." THE JOURNAL OF EXPERIMENTAL MEDICINE 7 AUG 2006, vol. 203, no. 8, 7 August 2006 (2006-08-07), pages 1875-1881, XP002432849 ISSN: 0022-1007
- BELLADONNA ET AL: "IL-23 neutralization protects mice from Gram-negative endotoxic shock" CYTOKINE, ACADEMIC PRESS LTD, PHILADELPHIA, PA, US, vol. 34, no. 3-4, 21 May 2006 (2006-05-21), pages 161-169, XP005542187 ISSN: 1043-4666

**Description**

Introduction

Overwhelming infection with resultant multiple organ failure, which has been termed the 'sepsis syndrome' [1], is a devastating illness, and is a common Intensive Care Unit (ICU) admission diagnosis, with an incidence of 3 per 1000 population per annum [2]. The sepsis syndrome has been characterised as a dysregulation of inflammation in response to infection, with life threatening organ failure attributable to a combination of excessive cytokine mediated inflammation, disseminated coagulopathy and disruption of the integrity of micro-vascular endothelium [3].

Of particular importance to the hosts' response to an invading pathogen is the induction of different elements of the CD4[+] T helper cell population. The distal cytokine response to infection is regulated primarily by the induced CD4[+] T cell population, which in turn is determined by the antagonistic interaction of the cytokines Interleukin 12 (IL-12) and Interleukin 4 (IL-4). These cytokines induce naïve CD4[+] T cells to differentiate phenotypically into T Helper 1 (Th1) or Th2 cells respectively [4, 5]. Interleukin-6 (IL-6) also plays a role in Th1/Th2 differentiation promoting Th2 differentiation primarily by inducing IL-4 production, whilst inhibiting Th1 differentiation by an IL-4 independent mechanism [6].

The Th1 cellular response is generally induced by, and particularly effective against, intracellular pathogens and those that activate macrophages and natural killer (NK) cells. A Th1 response is associated with enhanced cell-mediated immunity and is therefore the appropriate response expected in the face of most common septic insults. Lymphocytes demonstrating a Th1 response pattern characteristically produce interferon-gamma (IFNγ), a pleiotrophic cytokine which functions primarily in optimising the bactericidal activity of phagocytes.

In contrast Th2 biased responses are classically responsible for defence against helminthic and arthropod infections and are an essential component of allergic type reactions. Mature Th2 cells preferentially secrete a number of cytokines, including Interleukin 10 (IL-10). The antibodies stimulated by this response, however, do not promote phagocytosis or activate complement efficiently.

WO 2004/108957 discloses a method for diagnosis and/or prognosis of a septic syndrome in a human patient by determining the respective mRNA levels for a plurality of biological cytokines markers, among them TGF-alpha, IL-10, TGF-beta, IL-1beta, in a peripheral blood sample from a human patient.

There is a need to determine more clearly the cellular response to an infection and the factors which affect the response.

Statements of Invention

According to the invention, there is provided a method for predicting a human patients response to an infection, the method comprising determining an IL-23 mRNA value in a sample from the patient, and correlating the value with a likelihood that the patient will develop sepsis in response to infection, wherein a low IL-23 mRNA value correlates with a likelihood of the patient developing sepsis in response to infection, and high IL-23 mRNA value correlates with a likelihood that the patient will not develop sepsis in response to infection.

Suitably, the sample is peripheral blood mononuclear cells, wherein a low IL-23 mRNA value correlates with less than 1824 copies of IL-23 mRNA per 10 million copies of β-actin, and a high IL-23 mRNA value correlates with 1824 or more copies of IL-23 per 10 million copies of beta-actin.

In one aspect, the invention includes a step of further predicting the outcome of sepsis in a patient, the method comprising the steps of determining a ratio of IL-27:IL-23 mRNA values in a sample from the patient, wherein a ratio greater than 1.45 correlates with death and a ratio less than 1.45 correlates with survival.

Typically, the step of predicting outcome further includes determining a ratio of IL-10:IFN-γ mRNA values in a sample from the patient, wherein a ratio greater than 2.85 correlates with death and a ratio less than 2.85 correlates with survival.

Suitably, the method of predicting outcome comprises a scoring system in which a patient is assigned a score of 1 or 0 depending on wether the ratio of IL-27:IL-23 mRNA values is above or below 1.45, respectively, and a score of 1 or 0 depending on whether the ratio of IL-10:IFN-γ mRNA values is above or below 2.85, respectively, wherein a sum of 2 correlates with high risk of mortality due to sepsis, a sum of 0 correlates with a low risk of mortality due to sepsis, and 1 correlates with an intermediate risk of death due to sepsis.

In another aspect, the invention provides a method for stratifying a patient into a sepsis/non-sepsis group, the method comprising the steps of determining a ratio of IL-27:IL-23 mRNA values in a sample from the patient, wherein a ratio greater than 1.45 correlates with sepsis group, and a ratio less than 1.45 correlates with non-sepsis group.

In the methods of the invention, the IL-27, IL-23, IL-10, and IFN-γ my mRNA values are suitably represented by IL-27, IL-23, IL-10 and IFN-γ mRNA copy numbers, respectively, and in which the copy numbers are normalised against a housekeeping gene. In one embodiment, the housekeeping gene is β-Actin.

In a preferred embodiment of the invention, the samples from the human patient is mononuclear cells from a peripheral blood sample, or white cells isolated in the Buffy Coat layer of a peripheral blood sample.

In the method of the invention, DNA for IL-27, IL-23, IL-10, and. IFN- γ is preferable amplified and quantified as a surrogate for IL-27, IL-23, IL-10, and IFN- γ mRNA copy number, respectively.

Typically, the IL-27, IL-23, IL-10, and IFN- γ mRNA copy number is measured in absolute terms by reference to a calibration curve constructed from a standard sample of DIVA and normalised to a house keeping gene.

The assay of the present invention may be used in the design of pharmaceutical studies of patients with infection and or sepsis, in order to stratify patients by severity of illness, predict a likely outcome, and use this information in the study design and subsequent analysis.

The prediction of the likelihood of developing severe sepsis, or septic shock or critical illness in response to infection, may be based on a logistic regression analysis of cytokine mRNA, with a risk stratification based on the relative probabilities of developing and not developing critical illness (or sepsis, or severe sepsis or septic shock) in response to infection.

The risk of persistent septic shock versus the risk of resolution of septic shock may be based on a cluster analysis.

mRNA levels for the cytokines listed in the present invention and / or ratios between the various cytokine mRNA levels, as measured by real time PCR and normalised against a housekeeping gene and further calibrated against a reference curve derived from a cytokine PCR standard, other than those already stated may be used in the prediction of outcome in response to infection and outcome in patients with sepsis.

The accuracy and / or predictive capacity of this invention may be further improved by isolation of specific subgroups of white cells from peripheral blood samples and use of these cell subgroups in real time PCR assays. These cell groups include purified isolates of peripheral blood mononuclear cells, CD4+ T cells, Natural Killer cells and Natural Killer T Cells.

Brief Description of the Drawings

The invention will be more clearly understood from the following description of two embodiments thereof, given by way of example only with reference to the accompanying drawings in which: -

Fig. 1 is a graph showing the results of the logistic fit for a group of patients against the relative risk. Those patients likely to develop sepsis are indicated by the dot to the left of the curve while those who are unlikely to develop sepsis are shown by the asterisk to the right of the curve (Appendix 1 and 2 give the statistical analysis);

Fig. 2 is a diagram showing the risk of developing sepsis in response to infection increasing from 16% to 100% as the empiric score increase from 0 to 3;

Fig. 3 is a diagram showing the characterisation of Interlukin-10 mRNA levels as high or low in cases of sepsis and non sepsis (Appendix 3 gives the statistical analysis);

Fig. 4 is a graph showing the cut off limits for characterising Interlukin-10 as high or low (Appendix 4 gives the statistical analysis);

Fig. 5 is a graph showing the cut off limits for characterising Inteferon gamma mRNA levels as low and high (Appendix 5 gives the statistical analysis);

Fig. 6 is a diagram showing the characterisation of interferon gamma mRNA levels as high or low in cases of sepsis and non sepsis (Appendix 6 gives the statistical analysis);

Fig. 7 is a graph showing one-way analysis of IL-23 mRNA in cases of sepsis and non-sepsis (Appendix 7 gives the statistical analysis);

Fig. 8 is a mosaic plot of contingency analysis of low v high IL-23 by sepsis and non-sepsis (Appendix 8 gives the statistical analysis);

Fig. 9 is a graph showing one-way analysis of IL-23 mRNA by low v high IL-23 (Appendix 9 gives the statistical analysis);

Fig. 10 is a graph showing analysis of IL-27 mRNA by group (Appendix 10 gives the statistical analysis);

Fig. 11 is a graph showing analysis of IL-27 mRNA by low v high IL-27 (Appendix 11 gives the statistical analysis);

Fig. 12 is a mosaic plot of contingency analysis of low v high IL-27 by sepsis and non-sepsis (Appendix 12 gives the statistical analysis);

Fig. 13 is a mosaic plot of contingency analysis of score by Sepsis v Non Sepsis (Appendix 13 gives the statistical analysis);

Fig 14 is a mosaic plot of contingency analysis of Score IL-10, 23 and Interferon gamma By Sepsis v Non Sepsis (Appendix 14 gives the statistical analysis);

Fig. 15 is a graph showing analysis of the ration of IL-10 to Interferon gamma based on outcome of patients (Appendix 15 gives the statistical analysis);

Fig. 16 is a graph showing analysis of the ration of IL-27 to IL-23 based on outcome of patients (Appendix 16 gives the statistical analysis);

Fig. 17 is a mosaic plot of contingency analysis outcome by risk of mortality (Appendix 17 gives the statistical analysis);

Fig. 18 is a graph showing the temporal pattern of survival in patients with sepsis (Appendix 18 gives the statistical analysis);

Fig. 19 is a diagram showing analysis of outcome by mortality risk (Appendix 19 gives the statistical analysis);

Fig. 20 is a graph showing the temporal pattern of survival in patients with sepsis (Appendix 20 gives the statistical analysis);

Fig. 21 is a diagram showing the restriction map and multiple cloning site for pDNR-LIB vector;

Fig. 22 is a diagram of the MCS, multiple cloning site of Fig 21. The stuffer fragment is replaced by the IL27 cDNA insert. Unique restriction sites are shown in bold or in grey;

Fig. 23a shows an RNA gel with 1kb ladder and plasmid obtained after miniprep; and

Fig. 23b shows an RNA gel with 1kb ladder and digested plasmid preparation.

## Detailed Description of the Invention

By using Quantitative real-time polymerase chain reaction (QRT-PCR) we have found a pattern of cytokine gene expression associated with both severe sepsis and with infection in the absence of critical illness. We have also been able to determine whether there was a characteristic cytokine response pattern linked to the occurrence and resolution of septic shock.

We found that the patient response to infection was associated with distinct patterns of cytokine mRNA production. Whereas Interferon gamma (IFN$\gamma$) mRNA, the signature cytokine for the Th1 response, was greatest in patients who tolerated infection with relative impunity, lesser IFN$\gamma$ mRNA was associated with both the occurrence of severe sepsis in response to infection and also with poor outcome, that is excess mortality and persistence of septic shock, in these ICU patients. Thus IFN$\gamma$ mRNA levels can be used to predict the occurrence of sepsis , or the non occurrence of sepsis, in response to infection. IFN$\gamma$ mRNA levels can also be used to predict outcome in patients who develop sepsis, as lesser IFN$\gamma$ mRNA is related to persistent shock and excess mortality rates.

In contrast, greater IL-10 mRNA levels were linked with the occurrence of severe sepsis in response to infection. As IL-10 is a Th2 cytokine, this indicates that the occurrence of severe sepsis may be linked to an imbalance in cytokine production secondary to infection, with a dominant Th1 response providing the optimal defence against infection.

The maturation of naïve CD4$^+$ T cells into the appropriate Th1 or Th2 phenotype is essential to launch an effective host response to a bacterial infection. Therefore, it is plausible that inter-individual variation in the gene expression of the Th1 inducing cytokines, IL-12 and IFN$\gamma$, the Th2 inducing cytokines IL-10 and IL-6, and the prototypic pro-inflammatory cytokine TNF$\alpha$, may be linked to both the clinical presentation and the subsequent outcome of patients with infections.

IL-12 mRNA levels were unrelated to the occurrence of severe sepsis, or to outcome in these ICU patients. This is somewhat surprising when one considers the importance of IL-12 in T cell differentiation. IL-12 is secreted predominantly by antigen presenting cells and acts on naïve CD4$^+$ cells, via the transcription factor STAT 4, to promote differentiation into a Th1, IFNγ producing, cell-type [7]. In animal models this effect is quite marked, with STAT 4 knockout mice displaying a deficit in bacterial clearance, which is partially reversed by exogenous IFNγ [8]. Curiously, the impaired immune response observed in this STAT 4 knockout model appears to be qualitative rather than quantitative, as the mice can mount an adequate leukocyte response to infection, but with inadequate bacterial clearance.

Furthermore, IL-12 therapy increases survival in animal models of sepsis [9]. A similar phenomenon has been observed in humans with sepsis, with lesser production of IL-12, specifically in response to endotoxin, predictive of death from severe sepsis after major surgery.

Surprisingly however, we found a lack of variability in IL-12 levels between groups indicating that, within the cytokine cascade, the basis for an inadequate Th1 response lies somewhere between IL-12 and IFNγ. However, from the data provided below, IL-12 mRNA may prove a useful marker in distinguishing patient response to infection in larger groups of patients, as IL-12 mRNA was greater in patients with infection who did not develop sepsis than in patients who develop sepsis in response to infection.

Although the ICU group had elevated TNFα mRNA levels in comparison to the controls, these levels were less than that observed in the bacteraemic group. The exaggerated TNFα response observed in patients with infection, in contrast to control volunteers, may represent an appropriate and protective response. This concept, that pro-inflammatory cytokines are beneficial to patients with infection, may in part account for the adverse outcome observed in prior studies of TNFα antagonists in patients with septic shock [10]. The discordant patterns of TNFα and IFNγ mRNA production observed between the ICU and the bacteraemic groups may be explained by the different cellular origins of these cytokines. TNFα is produced by a wide variety of cell types, primarily macrophages, whereas IFNγ is produced primarily by Th1 cells and NK cells. Alternatively, it is plausible that the reduction in IFNγ production may be a direct consequence of the increased levels of IL-6 observed in these patients, as IL-6 is a recognised inhibitor of Th1 differentiation [6].

QRT-PCR has recently been used to study cytokine gene expression in septic shock in a human study [11, 12]. The expression of the MHC class II genes were said to be down regulated in patients with sepsis and that non-survivors of septic shock had greater IL-10:IL-1 gene expression ratios. The study focused on the outcome of patients with septic shock, and did not examine the relation between IL-10 mRNA and the occurrence of sepsis in response to infection. Furthermore this study did not quantify or analyse IFNγ mRNA levels. We have quantified cDNA levels as a surrogate for specific mRNA by QRT-PCR thereby providing specific data for mRNA levels in a given sample.

We found an increase in IL-10 mRNA levels in septic patients when compared to controls. However we found that patients who are tolerant of infection do not produce excess IL-10 mRNA. However, in contrast to previous studies, we failed to observe a link between increase in mortality and excess IL-10 mRNA levels [11]. As IL-10 mRNA levels are greater in patients who develop sepsis in response to infection, and as IL-10 mRNA levels are not increased in patients with infection, then IL-10 mRNA levels can be used to predict response to infection in terms of the occurrence or non occurrence of sepsis in response to infection. Furthermore a combination of IFNγ and IL-10 levels can be used to accurately predict the occurrence or non concurrence of sepsis in response to infection. In addition , in patients with infection and with elevated IL-10 mRNA levels, then in these patients the therapeutic administration of an IL-10 antagonistic medication would be indicated for the prevention of subsequent occurrence of sepsis.

We found that greater IL-6 levels were associated with lesser TNFα and IFNγ mRNA levels in a patient group with poor outcome. IL-6 is a Th2 cytokine; it activates the acute phase response and controls switching of immunoglobulin subclasses. Despite these pro inflammatory actions IL-6 may not possess significant bactericidal activity, as witnessed by IL-6 knockout mice not experiencing excess mortality in animal models of peritonitis [13]. Yet IL-6 production is undoubtedly of importance in patients with sepsis, as high IL-6 levels are predictive of excess mortality, with certain IL-6 haplotypes linked with greater IL-6 production and greater severity of organ failure in patients with severe sepsis [14].

However, IL-6 also acts to modulate the balance between the Th1 and Th2 response to infection, promoting the Th2 response via both IL-4 dependant and independent mechanisms, and by inhibiting the Th1 response [6]. As IFNγ is the prototypic Th1 cytokine, with a pivotal role in generating cell mediated bactericidal activity, IL-6 may actually impair phagocytic bactericidal activity by inhibiting IFNγ production. This particular phenomenon is specifically evident in the context of mycobacterial disease where IL-6 has been demonstrated to inhibit IFNγ production and associated bactericidal activity [15]. Moreover, this phenomenon may represent the basis for the linkage of greater IL-6 protein levels with lesser TNFα and IFNγ mRNA observed in our patients with persistent shock and greater mortality.

Thus, IL-6 may generate excess inflammation while simultaneously impairing bactericidal activity. Thus a therapeutic intervention in patients with sepsis, severe sepsis and or septic shock, which aimed to antagonise the effects of interleukin 6 appears indicated in any general population of patients with sepsis, severe sepsis or septic shock, but would be specifically indicated in any subgroup of these patients with lesser IFNγ gene expression as assayed by mRNA levels and specified in this invention.

Interleukin 12 is of particular interest in the elaboration of a cytokine mediated inflammatory response to sepsis, as this cytokine regulates interferon gamma production by cells of both the innate and adaptive immune systems, and IL-12 gene deletion experiments indicate that IL-12 has an important role in the clearance of pathogenic bacteria. Furthermore IL-12 is a hetero-dimer, sharing a common p40 subunit with a family of the interleukins 18, 23 and 27, which may also be involved in the cytokine mediation of the immune response to sepsis.

IL-18 acts as a cofactor, along with IL-12, to induce Th1 development. Whilst not essential for Th1 differentiation, IL-18 optimises IFN$\gamma$ production, having a synergistic role in the presence of IL-12. However, the precise role of IL-18 in human sepsis remains unclear as it has also may act as or Th2 polarising cytokine in certain circumstances. The discovery that IL-18/IL-12 deficient mice retained the ability to produce Th1 cells indicated alternative pathways of Th1 development. This heralded the discovery of IL-23, a heterodimer of the p40 subunit of IL-12 and a p19 subunit, which functions similarly to IL-12 and induces IFN$\gamma$ activity. However, in contrast to IL-12, which acts primarily on naïve T cells, IL-23 induces proliferation of memory T cells and therefore promotes end stage inflammation. IL-27 is a cytokine structurally related to the IL-12 family, which was originally ascribed pro-inflammatory properties with Th1 inducing activity. Lastly some members of this family of cytokines are pleiotrophic, with IL-23 and IL-27 acting respectively to augment or repress cytokine production by CD4 cells with a Th17 phenotype.

We hypothesise that alterations in interferon gamma regulators other than IL-12 may prove important in the human response to infection. We demonstrate herein that the occurrence of sepsis and outcome from sepsis was linked with distinct patterns of mRNA production for, IL-23 and IL-27.

There are obvious therapeutic implications to the hypothesis that both severe sepsis and late septic shock are related to a relative pro-inflammatory cytokine deficiency. IFN$\gamma$ and IL-12 are commercially available and might be of benefit to patients who develop severe sepsis in response to an infective insult. The benefits may be more obvious in those patients who develop persistent shock from severe infection, particularly if documented insufficiency in IFN$\gamma$ gene expression is available. Many case series document the beneficial use of exogenous IFN$\gamma$ in leishmaniasis, leprosy and multi-drug resistant tuberculosis. The therapeutic use of IFN$\gamma$ in patients with sepsis has been less extensively reported. Two studies, one recruiting consecutive patients following traumatic injury and another recruiting patients after thermal injury failed to show any survival benefit from therapy with subcutaneous IFN$\gamma$ [16,17]. However, a case series of patients with severe sepsis coupled with documented monocyte suppression appeared to benefit from daily subcutaneous IFN$\gamma$ [18]. Similarly, trauma patients with diminished immune responsiveness, as measured by monocyte MHC class II receptor status, were less likely to develop nosocomial pneumonia when treated with inhaled IFN$\gamma$ [19].

As can be seen from this brief literature review the results of trials where Interferon gamma was used as a therapy in patients with sepsis are mixed. This may have a basis in inter individual difference in the interferon gamma gene expression as documented in this manuscript, with excess mortality observed in septic patients with deficient interferon gamma gene expression. As exogenous administration of Interferon gamma is unlikely to have any therapeutic effect in septic patients with appropriate interferon gamma gene expression, an assay, such as is documented in this manuscript which measured the degree of interferon gamma gene expression, may indicate subgroups of patients with sepsis who are most likely to benefit from exogenous administration of interferon gamma in a therapeutic regimen.

In the present invention we use the sensitivity of QRT-PCR as a method for evaluating cytokine gene expression. The QRT-PCR based mRNA assay of the invention provides unique *in vivo* information, which can be used as an index of the adequacy of the cytokine response to infection, and can be used to predict outcome in response to infection. Furthermore quantifying specific cytokine gene expression by measuring mRNA by real time polymerase chain reaction could be used as an indication for therapeutic antagonism of IL-6 and IL-10, and administration of exogenous IFN$\gamma$ in a therapeutic intervention.

We have found that measuring mRNA by QRT-PCR in patients with infection is a very useful diagnostic tool. We found a marked association between the pattern of cytokine gene expression and the occurrence of severe sepsis, persistent shock and survival. These results raise the intriguing possibility of treating patients with persistent shock and documented inhibition of IFN$\gamma$ gene expression with exogenous IFN$\gamma$. Similarly patients with sepsis and excess IL-6 and or IL-10 mRNA. could be treated with a therapeutic antagonist of these cytokines. These specific intervention could be used in isolation or as a combined therapy.

We found that cytokine mRNA levels may be used to differentiate between patients who tolerate infection with impunity, without developing the sepsis syndrome and those who are likely to develop the sepsis syndrome in response to infection. This prediction is based on the mRNA pattern for TNF$\alpha$, IFN$\gamma$, IL-10 IL-23 and IL27, with the mRNA measured by QRT-PCR and normalised to a house-keeping gene.

When the absolute values for mRNA copy numbers for these cytokines are used in a model to predict whether patients develop severe sepsis or tolerate infection, using a logistic regression analysis, the $R^2$ value of the model is 0.78, and receiver operator curve is 0.98. This model can be used to generate the respective probabilities of developing severe sepsis and of tolerating infection, and the ratio of these probabilities. This later ratio represents a scoring system for the risk of developing severe sepsis in response to infection.

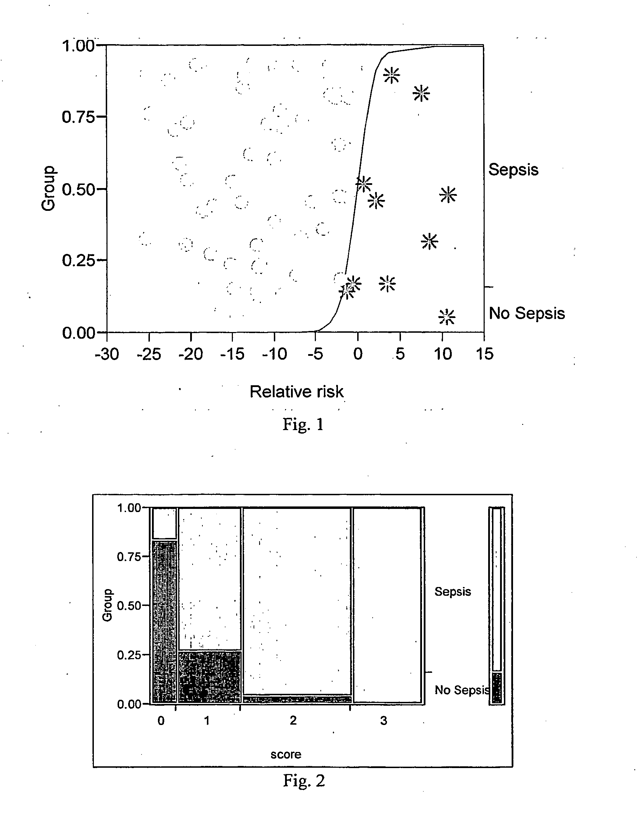

In a logistic regression analysis of this risk score against outcome, with outcome being either severe sepsis or tolerance to infection, the resulting logistic regression curve has 100% cut off points (Fig. 1). Thus, it is possible to select a score that can identify patients who will tolerate infection without developing severe sepsis with 100% specificity and with 70% sensitivity. Conversely, patients who develop severe sepsis in response to infection can be identified with 100% specificity and with 60% sensitivity.

**A Model to distinguish between Patients with Sepsis From Patients with Infection**

The scoring system described herein may be used to risk stratify patients who have infection, to identify patients who could either be treated with antibiotics for a short duration or who could receive therapy as outpatients. Alternatively, patients who are very likely to develop severe sepsis may be identified prior to the onset of a life threatening illness. These patients could receive appropriate intervention with antibiotics or surgical drainage of infected tissue.

In patients who already have developed critical illness in response to infection the mRNA pattern for the cytokines TNF$\alpha$ and IFN$\gamma$ may be used to identify patients who are at greater risk of developing a persistent shock. This risk stratification is based on a hierarchical cluster analysis, using Wards method with standardised data, and identifies a cluster of patients who experience persistent septic shock and greater severity of organ failure. This high risk cluster have a 10 fold increased risk of persistent septic shock, and might conceivably benefit from administration of exogenous IFN$\gamma$, to ameliorate the severity or shorten the duration of septic shock.

A further scoring system is shown in Table 1 giving the results of a statistical program used to provide standard values for TNF$\alpha$, IFN$\gamma$ and IL-10. When the values are applied to the population and the individuals categorised, or scored, on a 0 or 1 basis depending on whether they lie in the at risk category, then a simple scoring system may be devised, ranging from 0 to 3.

Table 1

| Count Row % | No Sepsis | Sepsis | |
|---|---|---|---|
| **0** | 5 83.33 | 1 16.67 | 6 |
| **1** | 4 26.67 | 11 73.33 | 15 |
| **2** | 1 4.00 | 24 96.00 | 25 |
| **3** | 0 0.00 | 16 100.00 | 16 |
| | 10 | 52 | 62 |

**Tests**

| Source | D | -LogLike | RSquare (U) |
|---|---|---|---|
| Model | 3 | 11.791109 | 0.4305 |
| Error | 58 | 15.600699 | |
| C. Total | 61 | 27.391808 | |
| N | 62 | | |

| Test | ChiSquare | Prob>ChiSq |
|---|---|---|
| Likelihood Ratio | 23.582 | <.0001 |
| Pearson | 27.059 | <.0001 |

Fig. 2 is a corresponding diagram showing the risk of developing sepsis in response to infection increasing from 16 to 100% as the empiric score increases from 0 to 3. The threshold values are, in terms of crossing threshold values as part of a real time polymerase chain reaction, standardised against a house keeping gene and referenced to a standard known quantity of mRNA for each respective cytokine, as listed below:

Point Values are in terms of mRNA copy numbers pre 10 million copies of $\beta$ Actin

TNF$\alpha$    0 if above 21380; 1 if below 21380

IL-10    1 if above 660; 0 if below 660

(continued)

IFNγ    0 if above 188; 1 if below 188

**Table 4** - The occurrence of sepsis by category of IL-10 and IFNγ mRNA.

| | High INF and Low IL-10 | Intermediate | Low INF and High IL-10 |
|---|---|---|---|
| **Sepsis** | 0 | 18 | 34 |
| **Combined Infection and Control groups** | 21 | 2 | 0 |
| **Total** | 21 | 20 | 34 |

INF = Interferon gamma.
IL-10 = Interleukin 10
High INF = > 230 copies mRNA
Low IL-10 is < 252 copies mRNA
R square = 0.5, Chi Square 79.5, p <0.0001.

**An Algorithm for the differentiation between patients with sepsis and all other patients.**

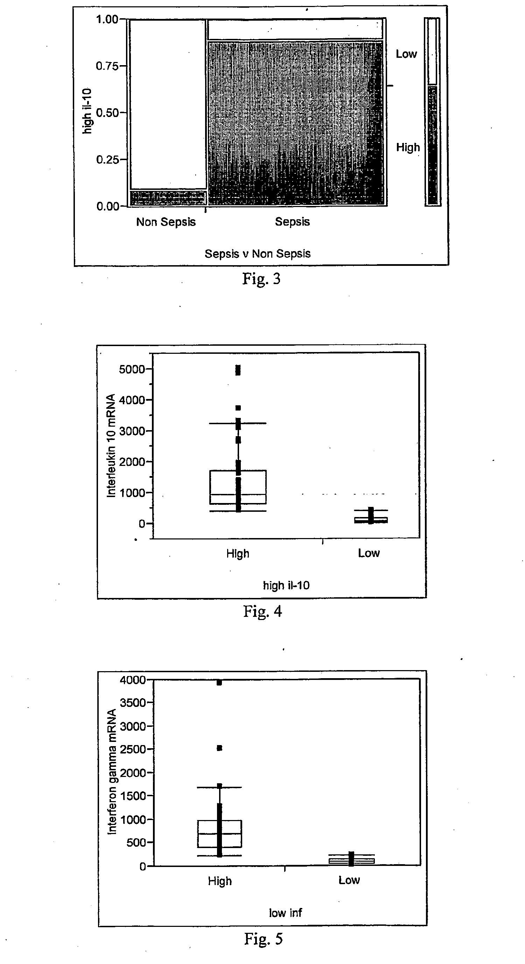

IL-10 mRNA can be used to differentiate between patients who develop sepsis and other patients by characterising IL-10 levels as either High or Low (Figure 3). Thus IL-10 mRNA levels can be characterised as High or Low IL-10 using the intervals shown in Figure 3.

The high and low categories of IL-10 have the limits as shown in Figure 4. Thus there is a cut point at 426 copies of IL-10 mRNA and the 95% confidence interval of this cut off is +/- 2 SEM of the difference between this cut off value and other values of IL-10 mRNA which is from 175 to 676 copies of IL-10 mRNA. (With all values of mRNA expressed per 10 million copies of β Actin)

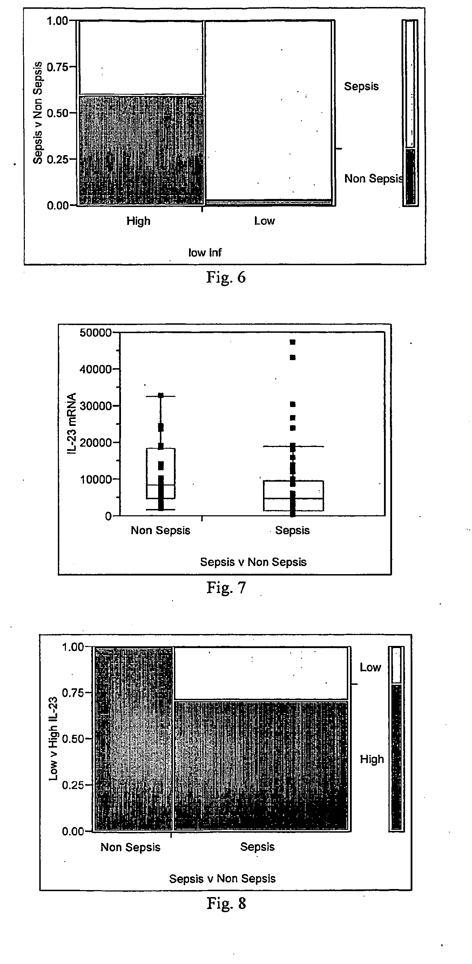

IFNγ mRNA levels can be used to distinguish between patients with sepsis and other patients by characterising IFNγ mRNA levels as high or low using the logistic regression analysis above. These categories of IFNγ mRNA have the limits as set out in Figure 5. Thus there is a cut off point at 240 copies of IFNγ mRNA per 10 million copies of β Actin and the 95% confidence interval of this cut off is from 100 copies to 380 copies. These categories of IFNγ mRNA can be used to distinguish between patients with sepsis and other patients, with low IFNγ observed with greater frequency in patients with sepsis (Figure 6).

**Interleukin 23**

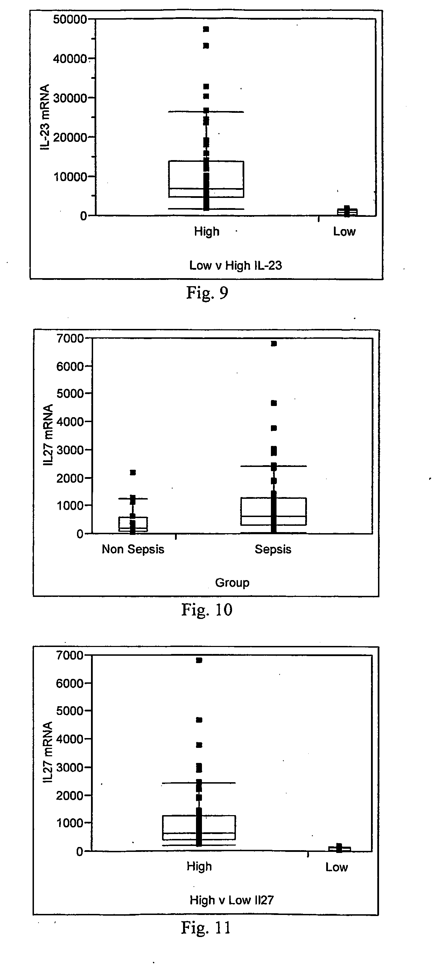

When patients with sepsis are compared with a combined group of healthy volunteers and patients with infection but without sepsis, then IL-23 is lower in patients with sepsis. Thus IL-23 may be of use in predicting response to infection. The graph of Figure 7 shows this data. When this data was partitioned into high and low IL-23 patients, with low being patients with less 1823.259 copies of Il-23 mRNA then all patients with IL-23 less than this value were in the sepsis group (as shown in Figure 8) The confidence internals of this characterisation of IL-23 are shown in Figure 9, it can be seen that there is a cut off point at 1824 copies of IL-23 mRNA per 10 million copies of β Actin and the 95% confidence interval of this cut off is from 4874 to 774 copies mRNA per million copies of β Actin. Thus IL-23 levels may be of use predicting outcome in response to infection.

**Interleukin 27**

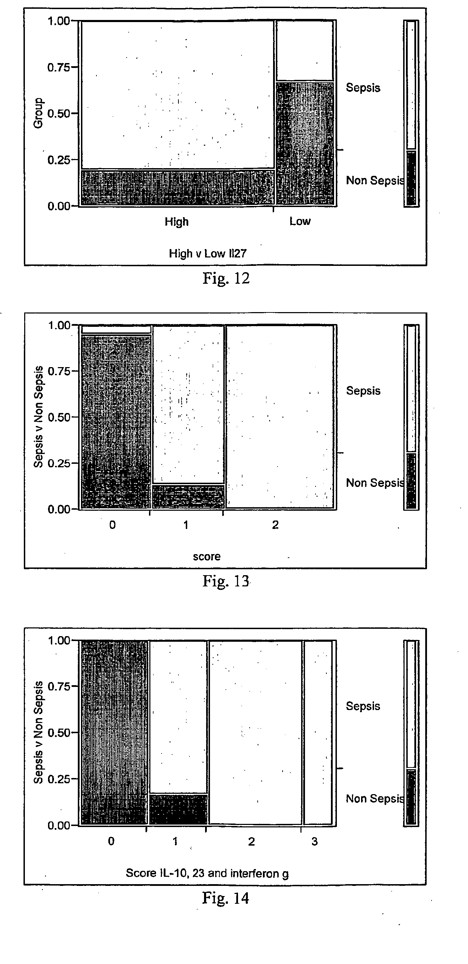

Referring to Figure 10, IL-27 mRNA was greater in patients with sepsis compared with other patients. IL-27 levels could be dichotomised by recursive partitioning into high and low categories as shown in Figure 11 There is a cut off point at 200 copies of IL-27 mRNA per 10 million copies of β Actin and the 95% confidence interval of this cut off is from 50 to 500 copies mRNA per 10 million copies of β Actin. This categorisation of IL-27 into high and low groups can be used to distinguish between patients with sepsis and other patients, as shown in Figure 12.

Thus IL-23 and IL-27 may be of use in predicting response to infection. However as both these cytokines regulate the CD4 Th17 cell line, then other cytokines which regulate this CD4 cell group such as IL-17 and Transforming growth factor beta -1 may be of use in predicting response to infection.

Referring to Figures 4, 5, 7, 9, 10, 11, 15 and 16, all values of mRNA are quoted as absolute copy numbers of mRNA with reference to a house keeping gene, which in this instance is β Actin, but other house keeping genes may be used.

**Scoring Systems to Distinguish between patients with Sepsis from all other Patients.**

A composite score is derived from the categoric values of IFNγ, IL- 10, 23 and 27:

Low IFNγ has an attributed value of 1 v 0 for the high category;

High IL- 10 has an attributed values of 1 v 0 for the low category;

Low IL- 23 has an attributed value of 1 v 0 for the high category; and

High IL-27 has an attributed value of 1 v 0 for the low category.

Summating these scores generates a cumulative value for risk of Sepsis. In a scoring system based on cumulative categoric values for IFNγ and IL-10 the resulting score, which ranges from 0 to 2, can be used to distinguish between patients with sepsis and other patients as shown in Figure 13.

In the scoring system based on Interleukin 10, 23 and IFNγ, the score ranges from 0 to 3 and can be used to distinguish patients with sepsis from other patients as shown in Figure 14.

**A model to predict outcome in patients with sepsis**

This same modelling system as described above can be used to predict outcome in patients who develop severe sepsis. This model is based on Cytokine mRNA levels in a blood sample drawn on the first day of admission to intensive care.

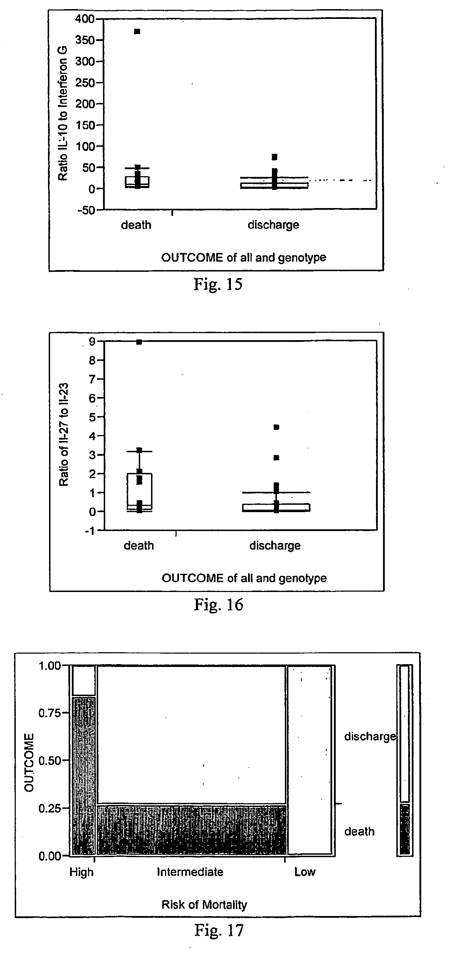

This scoring system is based on mRNA assays for the cytokines IL-10, 23 and 27 and IFNγ, on admission to intensive care, or in the initial phases of an episode of sepsis. In particular the combination of these molecules may be used to stratify patients. For example, in patients with sepsis death, sepsis is related to greater ratio of IL-10 : IFNγ mRNA, and also to greater ratio of IL-27 : IL-23, as is shown in Figures 15 and 16 respectively.

These ratios can be dichotomised into categories of high and low by recursive partitioning as shown in Tables 2 and 3 below.

**Table 2** - Quantiles for analysis of ratio IL-10 to Interferon Gamma by category

| Level | Minimum | 10% | 25% | Median | 75% | 90% | Maximum |
|---|---|---|---|---|---|---|---|
| High | 2.958275 | 3.258666 | 5.154364 | 10.12451 | 24.97909 | 51.41479 | 368.8188 |
| Low | 0.037236 | 0.100708 | 0.502283 | 1.045996 | 1.795874 | 2.745425 | 2.759358 |

Thus there is a cut off ratio of 2.85 and the 95% confidence interval of this ratio ranged from to 4.52 to 1.8.

**Table 3** - Quantiles for analysis of ratio of IL-27 to IL-23 by Category

| Level | Minimum | 10% | 25% | Median | 75% | 90% | Maximum |
|---|---|---|---|---|---|---|---|
| High | 1.523321 | 1.523321 | 1.76629 | 2.798216 | 4.444314 | 8.925306 | 8.925306 |
| Low | 0.001791 | 0.008563 | 0.049982 | 0.099343 | 0.337921 | 1.037605 | 1.383855 |

Thus there is a cut off ratio of 1.45 and the 95% confidence interval of this ratio ranged from to 2.6 to 0.13.

These categories (cut off ratio for IL-10 : IFNγ or IL-27 : IL-23 ) can be attributed (divided) into a "high" or "low" group depending on whether the cut off ratio is above or below the desired ratio (such as above or below about 1.45 for the IL-27 : IL-23 ratio or above or below about 2.85 for the IL-10 : IFNγ ratio). A score of 1 was attributed to a High value and a score of 0 was attributed to a Low value. When the two cut off ratios for IL-10 : IFNγ and IL-27 : IL-23 are combined a scoring system which ranges from 0 to 2 may be generated as follows:

| Score for IL-10 : IFNγ ratio | Score for IL-27 : IL-23 ratio | Total score for IL-10 : IFNγ and IL-27 : IL-23 ratio |
|---|---|---|
| 0 | 0 | 0 |
| 1 | 0 | 1 |
| 0 | 1 | 1 |
| 1 | 1 | 2 |

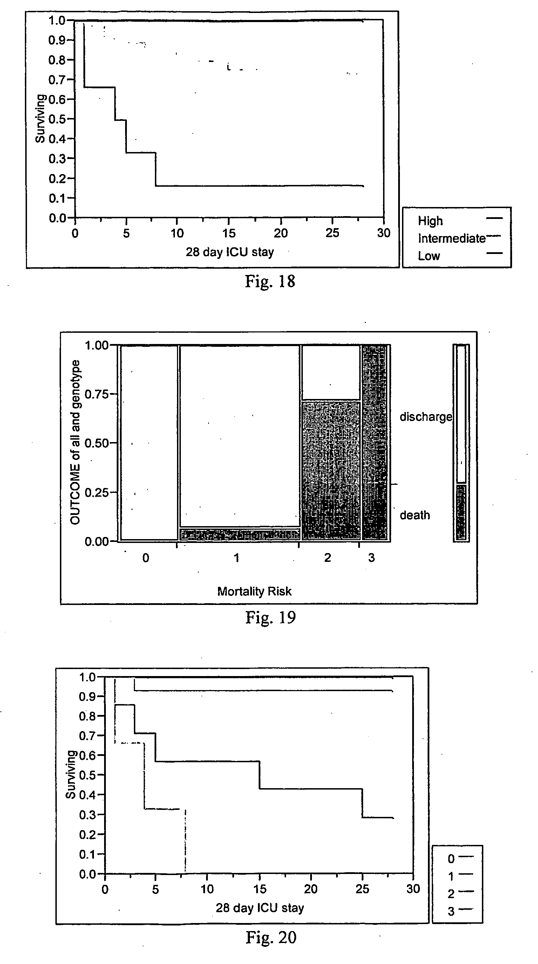

This scoring system may be used to predict risk of mortality, with a total score of 0 as low risk, 2 as high risk, and 1 an intermediate risk, as shown in Figure 17. Furthermore this scoring system can be used to predict the temporal pattern of survival in patients with sepsis (see Figure 18). These ratios, IL-10 : IFNγ and IL-27 : IL-23, have been given equal weighting in the scoring system, with a score of either 0 or 1 attributed to these (cytokine) ratios. However the relative importance of these ratios may not be equal. Thus the relative importance of these ratios may vary, and the relative importance or weighting of these ratios may not be equal, with one ratio having a weight of up to 10 times greater than the weight of other ratios.

The occurrence of sepsis by category of IL-10 and IFNγ mRNA can be seen in Table 4. Therefore the use of the IL-10 : IFNγ ratio and/or the IL-23 : IL-27 ratio may be useful in stratifying patients into sepsis / non-sepsis groups.

When Plasma levels of IL-6 protein are added to this model, and dichotomised as high, that is greater than 746pg/mL and attributed a risk score of 1, or Low, that is less than 746 pg/mL and attributed a risk score of 0, then this score can be added as an additional layer to the pre existing score. This generates a score, with range 0 to 3, which are linked to an increased risk of death from sepsis. Figure 19 illustrates this increased risk Furthermore this more complex risk score can be used to predict the temporal pattern of survival in patients with sepsis, as shown in Figure 20. Whilst the data for IL-6 presented herein relates to the concentration of IL-6 protein, it will be apparent to a person skilled in the art that the present invention also encompasses the use of IL-6 mRNA data.

The value of Outcome Prediction in patients with Sepsis and Infection.

The data presented in the preceding text outlines a methodology for predicting response to infection and or outcome in patients with severe sepsis. This methodology can be used as a tool to facilitate study design in an investigation of novel therapeutic interventions in patients with sepsis. As patients may be categorised by risk of mortality, specific patient groups, most likely to benefit from a specific therapy can be identified. On this basis patients with severe sepsis, but with low risk of mortality might be excluded from the study of a novel therapy, which may have a potential for toxicity.

In patients with sepsis, the information provided by this invention, in tandem with the potential benefit of a therapy, in terms of relative reduction in mortality rate, can be used to design a study of sufficient size so as to have sufficient power as to reach a statistically significant result. Thus instead of studying a general cohort of patients with sepsis, patients would be categorised by risk of mortality upon recruitment, allocated to a specific subgroup, with each sub-grouped sized to achieve statistical significance, and outcome for these subgroups analysed separately. On this basis, this invention can be used as a tool to facilitate logical patient recruitment and study design.

Furthermore the process of risk stratification for patients with sepsis outlined herein would be of use in identifying which patient groups would be most likely to benefit from any future novel therapies, could be used to monitor response to therapy, and could be used to identify patients who are more likely to experience injurious therapy related toxicity than to derive a therapy related benefit. This process is also applicable to the investigation of therapies for infection with novel anti microbial medications. This invention proposes an algorithm, based on cytokine mRNA, which predicts the occurrence of sepsis in patients with infection, and which may act as a covariate in the prediction of likely therapeutic failure or success.

Categorising patients according to the likelihood of response to a novel therapy maybe important for example for pharmaceutical companies as at present pharmaceutical companies bringing novel therapies for sepsis to the market lack an objective index which might differentiate between potential responders and non responders. Such an index or marker would have a major impact on the conduct of clinical trials of novel therapies in sepsis, and on the subsequent application of a novel therapy in clinical practice.

The invention will be more clearly understood from the following examples thereof.

Patients

This study was conducted in St James's Hospital, Dublin, Ireland, and was approved by the institutional ethics committee. Informed written consent was obtained from each patient or a relative. A total of 62 consecutive patients of Irish descent with severe sepsis or septic shock, as defined by the American College of Chest Physicians/Society of Critical Care Medicine Consensus Conference [1] were enrolled over the course of 12 months. All ICU patients received similar standardised care.

Severity of illness was characterised on admission to ICU using the Simplified Acute Physiology Score (SAPS2), Multiple Organ Dysfunction Score (MODS) and the Sequential Organ Failure Assessment (SOFA) scoring systems and again on day 7 with the MODS and SOFA scores.

Individual clinical and laboratory variables were collected on day 1 and day 7 of ICU stay. The recorded variables represented the most significant derangements from normal values recorded by the nursing staff over each 24-hour period. The duration of dialysis, inotrope dependence, ventilation and ICU stay was recorded for each patient. The source of infection necessitating the ICU admission, subsequent infections over the course of the ICU stay and the respective pathogenic organisms were noted. ICU death or survival to ICU discharge was recorded.

Ten consecutive patients with a gram negative bacteraemia, confirmed on blood culture, and with no organ failure or impending septic crisis were recruited from hospital wards. Thirteen healthy staff members served as a control group.

Exclusion Criteria

Exclusion criteria included: 1) infection with the human immunodeficiency virus; 2) patients neutropenic as a result of chemotherapy; 3) patients receiving long term treatment with corticosteroids; 4) patients receiving immunosuppressive therapy after organ transplantation; 5) non-Caucasian ethnic background.

Blood sampling

Blood sampling was carried out within the first 24 hours of ICU admission and again 7 days later through an indwelling central venous line or by venipuncture. In bacteraemic patients, blood sampling was carried out within 24 hours of the positive blood culture being reported. Blood samples were collected from healthy controls at one time point.
Peripheral Blood Mononuclear Cells (PBMCs) were immediately purified by density gradient centrifugation of EDTA anticoagulated blood using lymphoprep (Nycomed Pharma, Oslo, Norway). Buffy coat, the fraction of a centrifuged blood sample that contains most of the white blood cells can be distinguished after centrifugation. The Buffy coat layer is the layer between the layer of clear fluid (the plasma) and the layer of red fluid containing most of the red blood cells. This thin layer in between, the buffy coat, contains most of the white blood cells and platelets.
Serum was obtained from whole blood clotted for 30 minutes at room temperature and spun at 2500rpm for 10 minutes.

Total RNA extraction and Reverse Transcription

Total RNA was isolated from lysed PBMC using a commercially available kit (Qiagen RNeasy kit) following the manufacturers instructions. In order to avoid amplification of contaminating genomic DNA all samples were treated with RNase-free DNase (Qiagen) for 15 minutes. The quantity and purity of extracted RNA was measured with a spectrophotometer (Eppendorf BioPhotometer, Eppendorf AG, Hamburg, Germany). The quality of the extracted mRNA was verified on an Agilent 2100 Bioanalyzer using the RNA Nano LabChip kit (Agilent Technologies, Palto Alto,CA, USA).
Total RNA was then reverse transcribed as follows: 11.15$\mu$l of water containing 500ng of total RNA was first incubated at 65°C for 10 minutes. 18.85$\mu$l of the reverse transcription mix containing the following components were added: (1) 3$\mu$l 0.1M DTT; (2) 4.5$\mu$l Dimethyl Sulfoxide: (3) 2$\mu$l 100$\mu$M Random Primers (Invitrogen Corp, Carlsbad, CA, USA); (4) 1.25$\mu$l Moloney Murine Leukemia Virus Reverse Transcriptase (Invitrogen Corp); (5) 6$\mu$l 5X First Strand Buffer (Invitrogen Corp); (6) 1.5$\mu$l 4mM deoxynucleotide triphosphate mix (Promega Corp, Madison MI, USA); (7) 0.6$\mu$l RNasin (Promega Corp) 10U/$\mu$l. The samples were then incubated at 37°C for 1 hour.

Primers and probes

All primer and probes used in this study were synthesized at Applied Biosystems (Foster City, CA, USA). TNF$\alpha$ and IL10 primers and probes were obtained as a pre-customised mix (Assay ID for TNF$\alpha$ is Hs00174128_m1 and for IL10 is Hs00174086_m1). $\beta$-actin, IL12p35 and IFN$\gamma$ primers and probes were designed and customised as per Stordeur et al [11]. The oligonucleotides for real-time PCR are listed in Table A.

Table A

| mRNA targets (bp) | Oligonucleotides (5'→3')[a] (nM)[b] | Product size | Final concentration |
|---|---|---|---|
| $\beta$-actin | F976: GGATGCAGAAGGAGATCACTG | 90 | F300; R300 |
| | R1065: CGATCCACACGGAGTACTTG | | |
| | P997: 6Fam-CCCTGGCACCCAGCACAATG-Tamra-p | | |
| IFN-$\gamma$ | F464: CTAATTATTCGGTAACTGACTTGA | 75 | F600; R900 |
| | R538: ACAGTTCAGCCATCACTTGGA | | |
| | P491: 6Fam-TCCAACGCAAAGCAATACATGAAC-Tamra-p | | |
| IL12p35 | F212: CTCCTGGACCACCTCAGTTTG | 76 | F600; R900 |
| | R287: GGTGAAGGCATGGGAACATT | | |
| | P234: 6Fam-CCAGAAACCTCCCCGTGGCCA-Tamra-p | | |

(continued)

| mRNA targets (bp) | Oligonucleotides (5'→3')a (nM)b | Product size | Final concentration |
|---|---|---|---|
| TNFα | Supplied as a precustomised mix from Applied Biosystems | | F900; R900; P250 |
| IL10 | Supplied as a precustomised mix from Applied Biosystems | | F900; R900; P250 |

a F, R and P indicated forward and reverse primers and probes, respectively; numbers indicated the sequence position. b Final concentration of forward (F) and reverse (R) primers and probe (P).

IL-12 is a heterodimeric protein composed of two subunits, p40 and p35, encoded by unrelated genes. Neither subunit has biological activity alone, although a p40 homodimer may act as IL-12 antagonist [12]. Furthermore, IL-12 production by monocytes results in a 500-fold excess of p40 relative to the active heterodimmer [13]. Approximately 20-40% of the p40 in the serum of normal and endotoxin-treated mice is in the form of the homodimer [14]. As a consequence of this we chose to measure the p35 subunit as an index of the IL-12 heterodimer activity.

Real-Time PCR

The PCR reactions were carried out in an ABI Prism 7000 (Applied Biosytems). All reactions were performed either in triplicate or in duplicate. Thermocycling was carried out in a 20μl final volume containing: (1) water up to 20μl; (2) 10μl Mastermix (Applied Biosystems); (3) 1, 2 or 3 μl of 6 pmol/μl forward and reverse primers (final concentration 300,600 or 900nM, see Table 1); (5) 1μl of 4 pmol/μl Taqman Probe (final concentration 200nM) or 1μl of pre-customised primer/probe mix with default primer and probe concentrations (Table 1); (6) 0.8μl of the standard dilution or 2.4μl cDNA. After an initial denaturation step at 95°C for 10 minutes, temperature cycling was initiated. Each cycle consisted of 95°C for 15 seconds and 60°C for 60 seconds, the fluorescence being read at the end of this second step. In total, 40 cycles were performed.

Standard curves and expression of the results

The DNA standards for TNFα, IL-10, β-actin, IL12p35 and IFNγ consisted of a cloned PCR product that included the quantified amplicon prepared by PCR from a cDNA population containing the target mRNA. Information on these oligonucleotide standards is given in Table B.

Table B

| mRNA targets | Oligonucleotides (5'→3')a | Product size (bp) | Conditions for "classical" PCRb |
|---|---|---|---|
| IL10 | F296: TTTACCTGGAGAGGTGATG | 476 | A=56, Mg=1.5 |
| | R771: TTGGGCTTCTTTCTAAATCGT | | |
| TNFα | F83: ACCATGAGCACTGAAAGCAT | 406 | A=58, Mg=1.5 |
| | R488: AGATGAGGTACAGGCCCTCT | | |
| IFNγ | F154: TTGGGGTTCTCTTGGCTGTTA | 479 | A=58, Mg=1.5 |
| | R632: AAATATTGCAGGCAGGACAA | | |
| β-actin | F745: CCCTGGAGAAGAGCTACGA | 509 | A=58, Mg=1.5 |
| | R1253: TAAAGCCATGCCAATCTCAT | | |
| IL12 | F185: AGCCTCCTCCTTGTGGCTA | 228 | A=59, Mg=1.5 |
| | R412: TGTGCTGGTTTTATCTTTTGTG | | |

a F and R indicate forward and reverse primers, respectively; numbers indicate the sequence position.
b Conditions, for all targets, were as follows: denaturation at 95°C for 20s, annealing (temperature as stated (A)) for 20s and elongation at 72°C for 45s, for a total of 35 cycles. MHCl2 concentration (Mg,mM) was as stated.

In order to quantity transcript levels a standard curve was constructed, for each PCR run, for each selected mRNA target from serial dilutions of the relevant standard. All standard curves showed correlation coefficients >0.99, indicating a precise log linear relationship.

The mean efficiency of the standard curves for all target cDNA was 98.75% +/-4.4%. The mRNA copy numbers were then calculated for each patient sample using the standard curve to convert the obtained crossing threshold (Ct) value into mRNA copy numbers. Results were then expressed in absolute copy numbers after normalisation against β-actin mRNA (mRNA copy numbers of cytokine mRNA per 10 million β-actin mRNA copy numbers).

Cytokine Enzyme Linked Immunosorbant Assay (ELISA)

Serum TNFα, IL10, IL6 and IFNγ concentrations were measured by ELISA (R+D systems, Minneapolis, MN, USA) following the manufacturers instructions. The lower limit of detection for TNFα was 15.625pg/ml, for IL10 was 46.875pg/ml, for IL6 was 9.375pg/ml and for IFNγ was 15.625pg/ml. All samples were tested in duplicate.

Statistical analysis

The Wilcoxon rank sum test was used to analyse the differences between groups for continuous variables. Change in a continuous variable over time was analysed by ANOVA for repeated measures. Categorical variables were analysed by Chi Square test and Fishers exact test as appropriate. Spearman's rank correlation coefficient was used to analyse the relationship between continuous variables. Hierarchical cluster analysis was performed with standardised data using Ward's method the results of which were analysed using the JMP statistical software package (SAS Institute, Cary North Carolina, US).

Real Time Polymerase Chain Reaction Methods

Cytokine mRNA was measured as an absolute value of copies of mRNA for respective cytokines, and from a single blood sample. This measurement was accomplished by using DNA standards, containing known quantities of DNA, and constructing a reference calibration RT PCR curve by the use of sequential dilutions of these standards.

Similarly reference curves for a house keeping gene, in our case □actin was constructed so that the cytokine mRNA measure could be normalised against this house keeping gene.

The reference PCR curve which was used as a calibration tool and permitted measurement of cytokine mRNA in absolute terms as copy numbers per million copy numbers of a house keeping gene.

Preparation of DNA standards

Pre-prepared cytokine standards

The DNA standards for TNFα, IL10, B-actin, IL12p35, IFNγ, IL18, IL4 and IL23p19 consisted of a cloned PCR product that included the quantified amplicon that was prepared by PCR from a cDNA population containing the target mRNA. Sequences and reaction conditions are given in Table C.

Stock solutions of standards, containing $10^9$ (IFNγ, B-actin, TNFα, IL23p35 and IL4) or $10^{10}$ (IL12p35 and IL10) copy numbers per μl, were aliquoted and stored at - 20°C. A dilution series from $10^9$ to $10^2$ copy numbers per μl was prepared in each case and stored at 4°C. The standards were diluted in TE buffer (10mM Tris-HCl, 1mM EDTA, pH 8.0) containing double-stranded herring DNA (Sigma) at 10μg/ml.

Table C - Oligonucleotides for standard preparation

| mRNA targets | Oligonucleotides (5'→3')[a] | Product size (bp) | Conditions for "classical" PCR[b] |
|---|---|---|---|
| IL10 | F296: TTTACCTGGAGAGGTGATG R771: TTGGGCTTCTTTCTAAATCGT | 476 | A=56, Mg=1.5 |
| TNFα | F83: ACCATGAGCACTGAAAGCAT R488: AGATGAGGTACAGGCCCTCT | 406 | A=58, Mg=1.5 |
| IFNγ | F154: TTGGGTTCTCTTGGCTGTTA R632: AAATATTGCAGGCAGGACAA | 479 | A=58, Mg=1.5 |
| B-actin | F745: CCCTGGAGAAGAGCTACGA R1253: TAAAGCCATGCCAATCTCAT | 509 | A=58, Mg=1.5 |
| IL12 | F185: AGCCTCCTCCTTGTGGCTA R412: TGTGCTGGTTTTATCTTTTGTG | 228 | A=58, Mg=1.5 |

(continued)

| mRNA targets | Oligonucleotides (5'→3')[a] | Product size (bp) | Conditions for "classical" PCR[b] |
|---|---|---|---|
| IL18 | F133: AGTCTACACAGCTTCGGGAAGA R653: GTCCTGGGACACTTCTCTGAAA | 113 | A=60, Mg=1.5 |
| IL4 | F27: TAATTGCCTCACATTGTCACT R52P: ATTCAGCTCGAACACTTTGAA | 503 | A=58,Mg=1.5 |

[a] F and R indicate forward and reverse primers, respectively; numbers indicate the sequence position.
[b] Conditions, for all targets, were as follows: denaturation at 95°C for 20s, annealing (temperature as stated (A)) for 20s and elongation at 72°C for 45s, for a total of 35 cycles. MgCl2 concentration (Mg,mM) was as stated.(For the complete procedure see Stordeur et al., 1995, PCR for IFNγ[125].

Preparation of the IL27p28 standard

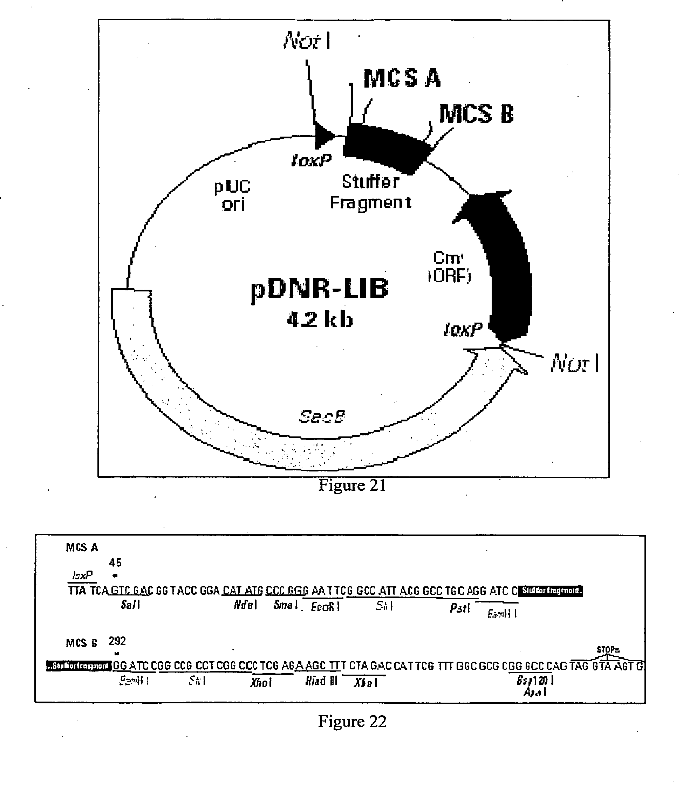

An IL 27 standard was fabricated de novo, which illustrates the methodology of constructing these standards.
IL27p28 plasmid purchased from Open Biosystems. It consisted of a 1.9kb cDNA clone inserted into a 4.2 kb vector transformed into chloramphenicol resistant E Coli. The vector was pDNR-LIB as shown in Figure 21. Figure 22 shows the MCS, multiple cloning site. The stuffer fragment is replaced by the IL27 cDNA insert. Unique restriction sites are shown in bold or in grey.
Plasmid DNA was cultured by being double-streaked on a chloramphenicol LB agar plate. A sample from this was then placed in a liquid culture overnight. Small and large scale plasmid DNA isolation was performed using Qiagen Mini and Midi kits, respectively. Restriction digestion procedures were performed by the methods of Sambrook et al. Following electorphoretic separation, DNA fragments were purified from gels using the QIAquick Gel Extraction Kit (Qiagen).

RNA Gels

All RNA gels were constructed by dissolving agarose in Tris-Borate-EDTA (TBE) buffer (Sigma). This was achieved by heating the mixture in a microwave to create a 1% agarose solution. Ethidium bromide was added to the gel at a final concentration of $0.5\mu$g/ml to facilitate visualisation of the DNA. The gel is then poured into a casting container containing a sample comb and allowed to cool to room temperature. After the gel has solidified, the comb is removed. The gel, still in its plastic tray, is inserted horizontally into the electrophoresis chamber and just covered with buffer. A voltage of 135V was then applied across to the gel for 35 minutes. An ultraviolet light box was then used to visualize ethidium bromide-stained DNA in gels.

Gel purification

The IL27 fragment was extracted from the agarose gel using a QIAquick Gel Extraction Kit (Qiagen) following the manufacturers instructions. The band of interest is carefully removed from the gel using a scalpel. This is then placed in a spin-column which contains a DNA binding silica-gel membrane. DNA adsorbs to this membrane in the presence of high salt buffer while contaminants pass through the column as impurities are washed away. Tris elution buffer is added to the center of the QIAquick membrane and the column is left to stand for 1 minute before the DNA is eluted with the elution buffer. This increases DNA yield by up to 1.7 times. Nucleic Acid quantification then took place using the nanodrop.

Determining the volume of plasmid DNA corresponding to copy numbers of target nucleic acid sequences

First we calculate the mass of a single plasmid molecule. Insert the plasmid size value into the formula below:

$$m= (n)(1.096 \times 10^{-21} \text{ g/bp});$$

where m=mass and n= plasmid size (bp), where the size of the entire plasmid (plasmid + insert) is used in this calculation. In the case of IL27:

$$m = 5277 \text{ bp}(1.096 \times 10^{-21}) \text{ g/bp}$$

$$= 5.78 \times 10^{-18} \text{ g} = \text{mass of 1 plasmid molecule}$$

We then calculate the mass of plasmid containing the CN of interest, in this example $10^{-11}$ copies:

$$10^{-11} \text{ copies} \times 5.78 \times 10^{-18} \text{ g/ copy}$$

$$5.78 \times 10^{-7} \text{ g}$$

We then calculate the concentrations of plasmid DNA needed to achieve the CNs of interest. We divide the mass needed by the volume to be pipetted into each reaction. For this example $5\mu L$ of plasmid DNA solution is pipetted into each PCR reaction. For $10^{-11}$ copies;

$$5.78 \times 10^{-7} \text{ g} / 5\mu L = 1.16 \times 10^{-7} \text{ g/}\mu L.$$

We then prepare a serial dilution of the plasmid DNA. We use the following formula to calculate the volume needed to prepare the $10^{-11}$ copy standard dilution. For this example the stock solution of plasmid DNA was taken as having a concentration $1.5\mu g/\mu L$ as determined by spectrophotometric analysis.

$$C_1 V_1 = C_2 V_2$$

$$(1.5\mu g/\mu L)(V_1) = (1.16 \times 10^{-7} \text{ g/}\mu L)(100\mu L)$$

$$V_1 = 7.73\mu L$$

For $10^{11}$ CN IL27 / $\mu L$ add 7.73 $\mu L$ to 92.27$\mu L$ of dilutent and from this point prepare a serial dilution of the plasmid DNA.

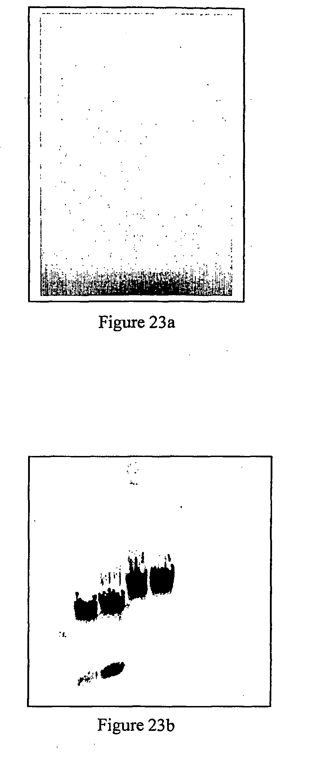

Figure 23 a shows an RNA gel with 1kb ladder and plasmid obtained after miniprep and Figure 23b shows an RNA gel with 1kb ladder and digested plasmid preparation.

Preparation of the IKBL standard

The Human IKBL cDNA was purchased from Invitrogen (H-X77909-M Invitrogen clone). The complete coding sequence was amplified using the following primers that inserted the restriction sites Nde1 and Xho 1:

-   Forward primer: ACTCAGATCATATGAGTAACCCCTC
-   Reverse Primer: TGGATCCTCGAGTCACGGTACCTTGAG

The reaction conditions for the PCR were 95°C for 15 min; 95°C for 30 s; 58°C for 1 min; 72°C for 1 min for 35 cycles.

The amplified products were gel purified using a Qiagen gel purification kit, confirmed by sequencing (Dept. of Biochemistry, Cambridge University, UK) and quantified using Pico green (Cambridge Biosciences, UK).

Results

Sixty-two ICU patients, 10 bacteraemic ward patients and 13 healthy controls were consented and recruited into the study. Blood samples were available for PCR analysis from 52 of these ICU patients on the first day of critical illness and from 49 ICU patients on the seventh day of critical illness. A total of 39 of the ICU patients had blood samples available for PCR analysis at both time points. Serum was collected for ELISA on 37 ICU patients on day 1 and 36 ICU patients on day 7. The illness severity scores and sites of infection for the ICU group are listed in Table 5.

**Table 5**

|  | Survivors | Non survivors | p |
| --- | --- | --- | --- |
| N | 45 | 17 |  |
| Male | 24 (53%) | 10 (59%) | ns |
| Age (years) | 62 (19-81) | 67 (39-86) | ns |
| SOFA day 1 | 7 (4-10) | 9 (9-13) | 0.003 |
| SAPS II | 40 (33-51) | 49 (41-60) | 0.02 |
| Ventilated Day 1 | 36 (80%) | 17 (100%) | ns |
| Inotropes Day 1 | 26(58%) | 16(94%) | 0.006 |
| Intensive Care Stay | 12 (3.5-27) | 5 (3 - 10.5) | 0.03 |
|  |  |  |  |
| Site of infection |  |  |  |
| Respiratory | 22 (49%) | 10 (59%) | ns |
| Abdominal | 15 (33%) | 5 (29%) | ns |
| Others | 8 (18%) | 2 (12%) | ns |

Results are presented both for the complete cohort of ICU patient. Age and severity of illness scores are presented as means with the range in parenthesis.

Table 6 shows the Cytokine mRNA quantification in Intensive Care Patients with Sepsis on Day 1 compared with Controls and Patients with Bacteraemia. On the first day of critical illness the ICU group showed greater TNF$\alpha$ and IL-10 mRNA levels and lesser IFN$\gamma$ mRNA levels than the control group. The ICU group had lesser TNF$\alpha$ and INF$\gamma$ and greater IL-10 mRNA levels in comparison with the bacteraemia group (Table 6). There was no difference in IL-12 mRNA levels between groups.

**Table 6** - Cytokine mRNA quantification in Intensive Care Patients with Sepsis on Day 1 compared with Controls and Patients with Bacteraemi

|  | ICU Day 1 | Bacteraemia | Control |
| --- | --- | --- | --- |
| N | 52 | 10 | 13 |
| TNF$\alpha$ | 19866 (11545-28270) | 36294 (28858-72275) | 577 (422-1679) |
| p |  | 0.0007 | <0.0001 |
| IL-10 | 851 (573-1638) | 163 (88-363) | 66 (33-81) |
| p |  | <0.0001 | <0.0001 |
| IL-12 | 2170(923-4039) | 3843 (1937-5910) | 2123(1676-5597) |
| p |  | 0.03 | ns[a] |
| IFN$\gamma$ | 131 (76-350) | 745 (285-941) | 883 (617-1216) |
| p |  | 0.0003 | <0.0001 |

(continued)

| | ICU Day 1 | Bacteraemia | Control |
|---|---|---|---|
| IL-4 N | 39 | 9 | 12 |
| | 9 (2-28) | 201 (22-512) | 161 (58-262) |
| p | | 0.005 | 0.0004 |

Results are expressed as mRNA copy numbers per 10 million β actin mRNA copies. All values are median and inter quartile range. All comparisons are by Wilcoxon rank sum test with p values stated as uncorrected values. All p values are for comparison with the ICU group.
[a] non significant

Table 7 shows the relation between cytokine mRNA levels and requirement for inotrope infusions on day 7 in Intensive Care Patients with sepsis. In the ICU group, there was no association between the cytokine mRNA levels and the presence or absence of shock on day 1 of critical illness. However on the seventh day, shocked patients displayed significantly lower TNFα and IFNγ mRNA levels than the patients without shock.

**Table 7** - The relationship between cytokine mRNA levels and the presence of shock, on day 7 in the ICU group.

| Cytokine | Shock Group | Group without Shock | p |
|---|---|---|---|
| N | 15 | 34 | |
| TNF | 9667 (6207-17291) | 19179 (10902-28543 | 0.001 |
| IL-10 | 355 (154-452) | 189 (56 - 312) | 0.11 |
| IL-12 | 2619 (2109-8014) | 4619 (2669-6699) | ns[a] |
| IFNγ | 150 (70 - 195) | 368 (196 - 772) | 0.0004 |
| IL-4 | 15 | 31 | |
| N | 229 (26 - 671) | 66 (15 - 270) | ns |

Results are expressed as mRNA copy numbers per million β actin mRNA copies.
All comparisons are by Wilcoxon rank sum test. All values are stated as median with inter quartile ranges.
[a] non significant

Results are expressed as log mRNA copy numbers per $10^7$ β actin mRNA copies. All comparisons are by Wilcoxon rank sum test. All values are stated as median with inter quartile ranges.

Seventeen of the 62 ICU patients (27%) died during the course of their ICU stay. Of the 52 patients that were analysed for mRNA production on the first day of critical illness, 13 died prior to ICU discharge. There was a non-significant trend towards greater IL-10 mRNA levels (median 3.10, inter quartile range 2.86 - 3.5 versus, median 2.89, inter quartile range 2.73 - 3.13; p = 0.1) and lesser IFNγ mRNA levels (median 2.05, inter quartile range 1.82 - 2.15 versus median 2.26, interquartile range 1.88 - 2.61; p = 0.07, all values expressed as mRNA copy numbers per 10 million β-actin copy numbers) in non-survivors when compared with survivors. However, when ICU patients were dichotomised between those in the highest quartile of IFNγ mRNA production on day 1 and the remainder, no deaths occurred in the highest quartile group, whereas 13 of the other 39 patients died (p = 0.02).

Table 8 shows the relationship between cytokine mRNA levels in Intensive Care Patients on day 7 of critical illness and subsequent outcome. Eight of the 49 ICU patients who had blood samples analysed for mRNA production after 7 days of critical illness died prior to ICU discharge. These 8 non-survivors had lesser TNFα mRNA levels and a trend towards lesser IL-12 and IL-10 mRNA levels in comparison to survivors.

**Table 8** - Relationship between cytokine mRNA levels in the ICU group on day 7 of critical illness and subsequent outcome.

| | Survivors | Non Survivors | p |
|---|---|---|---|
| N | 41 | 8 | |
| TNFα | 17329 (10766-25600) | 6898 (3814-9605) | 0.002 |
| IL-10 | 255 (133-402) | 40 (24 - 348) | 0.08 |
| IL-12 | 4672 (2619-6885) | 2420(1588-6338) | ns[a] |
| INFγ | 293 (158-677) | 148 (59-565) | ns |

(continued)

| | Survivors | Non Survivors | p |
|---|---|---|---|
| IL-4 N | 38 | 8 | |
| | 76 (27 - 281) | 178(10 - 954) | ns |

Results are expressed as mRNA copy numbers per 10 million β actin mRNA copies. All values are median and inter quartile range. All comparisons by Wilcoxon rank sum test with p values stated as uncorrected values.

Results are expressed as log mRNA copy numbers per $10^7$ □ actin mRNA copies. All values are median and inter quartile range. All comparisons with control by Wilcoxon rank sum test with p values stated as uncorrected values.

A cluster analysis, which included ICU data for day 7 TNFα and IFNγ mRNA levels, was performed and a cluster of ICU patients with reduced TNFα and IFNγ mRNA levels was characterised. Patients in this cluster were more likely to require inotropes on day 7 of critical illness, had greater SOFA scores and had a significantly higher mortality (Table 9).

**Table 9** - Cluster analysis of TNFα and IFNγ mRNA levels in the ICU group on Day 7 of critical illness

| Cluster | 1 | 2 | p |
|---|---|---|---|
| N | 31 | 18 | |
| TNFα | 21379 (16218-28840) | 6760 (2884-10715) | 0.0001 |
| INFγ | 407 (190-794) | 158 (93 – 229) | 0.0004 |
| Shock | 4 (13%) | 11 (61%) | 0.0009 |
| SOFA | 3 (0-6) | 10 (8-13) | 0.0001 |
| Outcome (Death) | 2 (6.5%) | 6 (33%) | 0.04 |

| IL-6 ( By Elisa in pg/ml) | | | |
|---|---|---|---|
| N | 21 | 15 | |
| IL-6 | 10.7 (0-103) | 142 (29 – 456) | 0.007 |

Results are expressed as log mRNA copy numbers per $10^7$ β actin mRNA copies (TNFα and IFNγ) or as pg/ml (IL-6). All continuous values are quoted as median with interquartile ranges. All comparisons are Wilcoxon Rank Sum test.

The majority of ICU patients had no detectable TNFα, IL-10 or IFNγ protein. However IL-6 was present in measurable quantities on day 1 (median 306, inter quartile range 131 to 731) and day 7 (median 63, inter quartile range 3.6 to 174, all values expressed as pg/ml). On the first day of critical illness there was a negative correlation between IL-6 levels and IFNγ mRNA levels (Spearman's Rho = -0.52, p=0.0009). On day 7 there was a negative correlation between IL-6 levels and TNFα mRNA levels (Spearman's Rho = -0.43, p=0.006). Also, on day 7, IL-6 protein levels were greater in ICU patients in the cluster with lesser TNFα and IFNγ mRNA production.

The data indicates that IL-6 acts differently to other pro-inflammatory cytokines and may antagonise a beneficial response resulting in adverse outcome. Based on the results it is envisaged that an anti-IL-6 antibody may potentially have use in conjunction with IFNγ in the preparation of a medicament for the treatment of patients with severe sepsis.

The invention is not limited to the embodiments hereinbefore described which may be varied in detail.

<u>**Appendix 1**</u>

**Nominal Logistic Fit for Group**

**Whole Model Test**

| Model | -LogLikelihood | DF | ChiSquare | Prob>ChiSq |
|---|---|---|---|---|
| Difference | 21.462100 | 1 | 42.9242 | <.0001 |
| Full | 5.929708 | | | |
| Reduced | 27.391808 | | | |
| | | | | |
| RSquare (U) | | 0.7835 | - | |
| Observations (or Sum Wgts) | | 62 | | |

Converged by Objective

**Lack Of Fit**

| Source | DF | -LogLikelihood | ChiSquare |
|---|---|---|---|
| Lack Of Fit | 60 | 5.9297076 | 11.85942 |
| Saturated | 61 | 0.0000000 | Prob>ChiSq |
| Fitted | 1 | 5.9297076 | 1.0000 |

**Parameter Estimates**

| Term | Estimate | Std Error | ChiSquare | Prob>ChiSq | Lower 95% | Upper 95% | Odds Ratio | Odds Lower | Odds Upper |
|---|---|---|---|---|---|---|---|---|---|
| Intercept | 1.50752e-7 | 0.809307 | 0.00 | 1.0000 | -1.5561144 | 1.88139339 | . | . | . |
| Relative risk | 1.00000023 | 0.4474755 | 4.99 | 0.0254 | 0.39721176 | 2.26336515 | 4.34883 e15 | 1628335 .94 | 2.48507e35 |

For log odds of No Sepsis/Sepsis

**Effect Wald Tests**

| Source | Nparm | DF | Wald ChiSquare | Prob>ChiSq |
|---|---|---|---|---|
| Relative risk | 1 | 1 | 4.99415089 | 0.0254 |

**Effect Likelihood Ratio Tests**

| Source | Nparm | DF | L-R ChiSquare | Prob>ChiSq |
|---|---|---|---|---|
| Relative risk | 1 | 1 | 42.9242 | 0.0000 |

## Appendix 2

**Whole Model Test**

| Model | -LogLikelihood | DF | ChiSquare | Prob>ChiSq |
|---|---|---|---|---|
| Difference | 21.462100 | 1 | 42.9242 | <.0001 |
| Full | 5.929708 | | | |
| Reduced | 27.391808 | | | |

| | |
|---|---|
| RSquare (U) | 0.7835 |
| Observations (or Sum Wgts) | 62 |

Converged by Objective

**Parameter Estimates**

| Term | Estimate | Std Error | ChiSquare | Prob>ChiSq |
|---|---|---|---|---|
| Intercept | 1.50752e-7 | 0.809307 | 0.00 | 1.0000 |
| Relative risk | 1.00000023 | 0.4474755 | 4.99 | 0.0254 |

For log odds of No Sepsis/Sepsis

**Receiver Operating Characteristic**

Area Under Curve =

**Appendix 3**

| Contingency Table<br>Sepsis v Non Sepsis By high IL-10 | | | |
|---|---|---|---|
| Count<br>Total %<br>Col %<br>Row % | High | Low | |
| **Non Sepsis** | 2<br>2.67<br>4.17<br>8.70 | 21<br>28.00<br>77.78<br>91.30 | 23<br>30.67 |
| **Sepsis** | 46<br>61.33<br>95.83<br>88.46 | 6<br>8.00<br>22.22<br>11.54 | 52<br>69.33 |
| | 48<br>64.00 | 27<br>36.00 | 75 |

**Tests**

| Source | DF | -LogLike | RSquare (U) |
|---|---|---|---|
| Model | 1 | 23.614651 | 0.4819 |
| Error | 73 | 25.391714 | |
| C. Total | 74 | 49.006365 | |
| N | 75 | | |

| Test | ChiSquare | | Prob>ChiSq |
|---|---|---|---|
| Likelihood Ratio | 47.229 | | <.0001 |
| Pearson | 44.037 | | <.0001 |
| Fisher's Exact Test | Prob | Alternative Hypothesis | |
| Left | <.0001 | Prob(high il-10=Low) is greater for Sepsis v Non Sepsis=Non Sepsis than Sepsis | |
| Right | 1.0000 | Prob(high il-10=Low) is greater for Sepsis v Non Sepsis=Sepsis than Non Sepsis | |
| 2-Tail | <.0001 | Prob(high il-10=Low) is different across Sepsis v Non Sepsis | |

**Appendix 4**

IL- 10 **Quantiles**

| Level | Minimum | 10% | 25% | Median | 75% | 90% | Maximum |
|---|---|---|---|---|---|---|---|
| High | 427.7401 | 517.0439 | 657.8845 | 949.4901 | 1710.892 | 3264.384 | 5004.377 |
| Low | 2.845719 | 13.31157 | 45.12425 | 89.35729 | 197.1628 | 347.0581 | 420.9414 |

There is a cut point at 426 copies of IL-10 mRNA and the 95% confidence interval of this cut off is +/- 2 SEM of the difference between this cut off value and other values of IL-10 mRNA which is from 175 to 676 copies of IL-10 mRNA. (With all values of mRNA expressed per 10 million copies of β Actin)

**Appendix 5**

**Interferon gamma Quantiles**

| Level | Minimum | 10% | 25% | Median | 75% | 90% | Maximum |
|---|---|---|---|---|---|---|---|
| High | 240.9588 | 304.9862 | 409.6467 | 691.7958 | 990.8418 | 1366.33 | 3917.509 |
| Low | 10.07806 | 25.09425 | 58.54774 | 104.3432 | 145.3965 | 200.1639 | 239.663 |

There is a cut off point at 240 copies of IFNγ mRNA per 10 million copies of β Actin and the 95% confidence interval of this cut off is from 100 copies to 380 copies.

**Appendix 6**

**Contingency Table**
Interferon gamma category By Sepsis By Non Sepsis

| Count<br>Total %<br>Col %<br>Row % | Non<br>Sepsis | Sepsis | |
|---|---|---|---|
| **High** | 22 | 15 | 37 |
| | 29.33 | 20.00 | 49.33 |
| | 95.65 | 28.85 | |
| | 59.46 | 40.54 | |
| **Low** | 1 | 37 | 38 |
| | 1.33 | 49.33 | 50.67 |
| | 4.35 | 71.15 | |
| | 2.63 | 97.37 | |
| | 23 | 52 | 75 |
| | 30.67 | 69.33 | |

**Tests**

| Source | DF | -LogLike | RSquare (U) |
|---|---|---|---|
| Model | 1 | 16.625981 | 0.3596 |
| Error | 73 | 29.604587 | |
| C. Total | 74 | 46.230568 | |
| N | 75 | | |

| Test | ChiSquare | | Prob>ChiSq |
|---|---|---|---|
| Likelihood Ratio | 33.252 | | <.0001 |
| Pearson | 28.473 | | <.0001 |
| Fisher's Exact Test | Prob | Alternative Hypothesis | |
| Left | 1.0000 | Prob(Sepsis v Non Sepsis=Sepsis) is greater for low inf=High than Low | |
| Right | <.0001 | Prob(Sepsis v Non Sepsis=Sepsis) is greater for low inf=Low than High | |
| 2-Tail | <.0001 | Prob(Sepsis v Non Sepsis=Sepsis) is different across low inf | |

**Appendix 7**

Quantiles for IL-23

| Level | Minimum | 10% | 25% | Median | 75% | 90% | Maximum |
|---|---|---|---|---|---|---|---|
| Non Sepsis | 1823.259 | 2747.308 | 4894.172 | 8414.014 | 18437.34 | 24025.62 | 32538.94 |
| Sepsis | 220.4026 | 655.6246 | 1561.336 | 4767.355 | 9487.687 | 22787.51 | 47140.12 |

**Wilcoxon / Kruskal-Wallis Tests (Rank Sums)**

| Level | Count | Score Sum | Score Mean | (Mean-Mean0/Std0 |
|---|---|---|---|---|
| Non Sepsis | 23 | 1090 | 47.3913 | 2.651 |
| Sepsis | 51 | 1685 | 33.0392 | -2.651 |

**2-Sample Test, Normal Approximation**

| S | Z | Prob>|Z| |
|---|---|---|
| 1090 | 2.65117 | 0.0080 |

**1-way Test, ChiSquare Approximation**

| ChiSquare | DF | Prob>ChiSq |
|---|---|---|
| 7.0597 | 1 | 0.0079 |

**Appendix 8**

| **Contingency Table** Sepsis v Non Sepsis By Low v High IL-23 | | | |
|---|---|---|---|
| Count Total % Col % Row % | High | Low | |
| **Non Sepsis** | 23 31.08 38.98 100.00 | 0 0.00 0.00 0.00 | 23 31.08 |
| **Sepsis** | 36 48.65 61.02 70.59 | 15 20.27 100.00 29.41 | 51 68.92 |
| | 59 79.73 | 15 20.27 | 74 |

**Tests**

| Source | DF | -LogLike | RSquare (U) |
|---|---|---|---|
| Model | 1 | 6.409682 | 0.1718 |

(continued)

**Tests**

| | | |
|---|---|---|
| Error | 72 | 30.895672 |
| C. Total | 73 | 37.305355 |
| N | 74 | |

| Test | ChiSquare | | Prob>ChiSq |
|---|---|---|---|
| Likelihood Ratio | 12.819 | | 0.0003 |
| Pearson | 8.485 | | 0.0036 |
| Fisher's Exact Test | Prob | Alternative Hypothesis | |
| Left | 1.0000 | Prob(Low v High IL-23=Low) is greater for Sepsis v Non Sepsis=Non Sepsis than Sepsis | |
| Right | 0.0017 | Prob(Low v High IL-23=Low) is greater for Sepsis v Non Sepsis=Sepsis than Non Sepsis | |
| 2-Tail | 0.0034 | Prob(Low v High IL-23=Low) is different across Sepsis v Non Sepsis | |

## Appendix 9

**IL-23 Quantiles**

| Level | Minimum | 10% | 25% | Median | 75% | 90% | Maximum |
|---|---|---|---|---|---|---|---|
| High | 1823.259 | 2370.565 | 4767.355 | 7096.964 | 14148.55 | 24424.69 | 47140.12 |
| Low | 220.4026 | 227.6223 | 420.7151 | 1018.412 | 1479.729 | 1654.136 | 1689.894 |

There is a cut off point at 1824 copies of IL-23 mRNA per 10 million copies of β Actin and the 95% confidence interval of this cut off is from 4874 to 774 copies mRNA per million copies of β Actin.

## Appendix 10

**IL-27 Quantiles**

| Level | Minimum | 10% | 25% | Median | 75% | 90% | Maximum |
|---|---|---|---|---|---|---|---|
| Non Sepsis | 19.26175 | 36.18736 | 80.8099 | 218.7316 | 598.7032 | 1248.712 | 2171.849 |
| Sepsis | 52.65335 | 173.9895 | 322.0002 | 623.8759 | 1312.294 | 2928.885 | 6782.582 |

**Wilcoxon / Kruskal-Wallis Tests (Rank Sums)**

| Level | Count | Score Sum | Score Mean | (Mean-Mean0)/Std0 |
|---|---|---|---|---|
| Non Sepsis | 20 | 460 | 23.0000 | -2.923 |
| Sepsis | 46 | 1751 | 38.0652 | 2.923 |

**2-Sample Test, Normal Approximation**

| S | Z | Prob>|Z| |
|---|---|---|
| 460 | -2.92310 | 0.0035 |

**1-way Test, ChiSquare Approximation**

| ChiSquare | DF | Prob>ChiSq |
|---|---|---|

(continued)

**1-way Test, ChiSquare Approximation**

| | | |
|---|---|---|
| 8.5853 | 1 | 0.0034 |

## Appendix 11

**IL-27 Quantiles**

| Level | Minimum | 10% | 25% | Median | 75% | 90% | Maximum |
|---|---|---|---|---|---|---|---|
| High | 202.8909 | 250.1165 | 390.5847 | 652.2764 | 1279.947 | 2890.477 | 6782.582 |
| Low | 19.26175 | 29.28958 | 51.60408 | 125.5221 | 151.6795 | 190.9291 | 192.0399 |

There is a cut off point at 200 copies of IL-27 mRNA per 10 million copies of β Actin and the 95% confidence interval of this cut off is from 50 to 500 copies mRNA per 10 million copies of β Actin

## Appendix 12

| **Contingency Table** High v Low IL-27 By Group | | | |
|---|---|---|---|
| Count Total % Col % Row % | Non Sepsis | Sepsis | |
| **High** | 10 15.15 50.00 19.61 | 41 62.12 89.13 80.39 | 51 77.27 |
| **Low** | 10 15.15 50.00 66.67 | 5 7.58 10.87 33.33 | 15 22.73 |
| | 20 30.30 | 46 69.70 | 66 |

**Tests**

| Source | DF | -LogLike | RSquare (U) |
|---|---|---|---|
| Model | 1 | 5.696549 | 0.1407 |
| Error | 64 | 34.788514 | |
| C. Total | 65 | 40.485063 | |
| N | 66 | | |

| Test | ChiSquare | Prob>ChiSq | |
|---|---|---|---|
| Likelihood Ratio | 11.393 | 0.0007 | |
| Pearson | 12.153 | 0.0005 | |
| Fisher's Exact Test | Prob | Alternative Hypothesis | |
| Left | 0.0011 | Prob(Group=Sepsis) is greater for High v Low Il27=High than Low | |

(continued)

| | | |
|---|---|---|
| Right | 0.9999 | Prob(Group=Sepsis) is greater for High v Low Il27=Low than High |
| 2-Tail | 0.0011 | Prob(Group=Sepsis) is different across High v Low Il27 |

**Appendix 13**

| **Contingency Table** Scoring system involving IL-10 and Interferon Gamma Sepsis v Non Sepsis By score | | | | |
|---|---|---|---|---|
| Count Total % Col % Row % | 0 | 1 | 2 | |
| **Non Sepsis** | 20 26.67 95.24 86.96 | 3 4.00 13.64 13.04 | 0 0.00 0.00 0.00 | 23 30.67 |
| **Sepsis** | 1 1.33 4.76 1.92 | 19 25.33 86.36 36.54 | 32 42.67 100.00 61.54 | 52 69.33 |
| | 21 28.00 | 22 29.33 | 32 42.67 | 75 |

**Tests**

| Source | DF | -LogLike | RSquare (U) |
|---|---|---|---|
| Model | 2 | 33.447486 | 0.4131 |
| Error | 71 | 47.522669 | |
| C. Total | 73 | 80.970154 | |
| N | 75 | | |

| Test | ChiSquare | Prob>ChiSq |
|---|---|---|
| Likelihood Ratio | 66.895 | <.0001 |
| Pearson | 58.335 | <.0001 |

**Appendix 14**

| **Contingency Table** Sepsis v Non Sepsis By Score IL-10, 23 and Interferon Gamma | | | | | |
|---|---|---|---|---|---|
| Count Total % Col % Row % | 0 | 1 | 2 | 3 | |

(continued)

| Contingency Table | | | | | |
| --- | --- | --- | --- | --- | --- |
| Sepsis v Non Sepsis By Score IL-10, 23 and Interferon Gamma | | | | | |
| **Non Sepsis** | 20 | 3 | 0 | 0 | 23 |
| | 26.67 | 4.00 | 0.00 | 0.00 | 30.67 |
| | 100.00 | 16.67 | 0.00 | 0.00 | |
| | 86.96 | 13.04 | 0.00 | 0.00 | |
| **Sepsis** | 0 | 15 | 28 | 9 | 52 |
| | 0.00 | 20.00 | 37.33 | 12.00 | 69.33 |
| | 0.00 | 83.33 | 100.00 | 100.00 | |
| | 0.00 | 28.85 | 53.85 | 17.31 | |
| | 20 | 18 | 28 | 9 | 75 |
| | 26.67 | 24.00 | 37.33 | 12.00 | |

**Tests**

| Source | DF | -LogLike | RSquare (U) |
| --- | --- | --- | --- |
| Model | 3 | 38.120466 | 0.3859 |
| Error | 69 | 60.673057 | |
| C. Total | 72 | 98.793524 | |
| N | 75 | | |

| Test | ChiSquare | Prob>ChiSq |
| --- | --- | --- |
| Likelihood Ratio | 76.241 | <.0001 |
| Pearson | 63.242 | <.0001 |

## Appendix 15

**Ratio of IL-10 to Interferon gama:Quantiles**

| Level | Minimum | 10% | 25% | Median | 75% | 90% | Maximum |
| --- | --- | --- | --- | --- | --- | --- | --- |
| death | 2.958275 | 3.049733 | 6.157191 | 10.39676 | 29.49803 | 240.9525 | 368.8188 |
| discharge | 0.037236 | 0.508411 | 1.627466 | 4.299748 | 13.83646 | 35.94041 | 74.05067 |

**Wilcoxon / Kruskal-Wallis Tests (Rank Sums)**

| Level | Count | Score Sum | Score Mean | (Mean-Mean0)/Std0 |
| --- | --- | --- | --- | --- |
| death | 13 | 452 | 34.7692 | 2.261 |
| discharge | 39 | 926 | 23.7436 | -2.261 |

**2-Sample Test, Normal Approximation**

| S | Z | Prob>|Z| |
| --- | --- | --- |
| 452 | 2.26117 | 0.0237 |

**1-way Test, ChiSquare Approximation**

| ChiSquare | DF | Prob>ChiSq |
| --- | --- | --- |

(continued)

**1-way Test, ChiSquare Approximation**

5.1608        1   0.0231

## Appendix 16

**Ratio of IL-27 to IL-23 :Quantiles**

| Level | Minimum | 10% | 25% | Median | 75% | 90% | Maximum |
|---|---|---|---|---|---|---|---|
| death | 0.049982 | 0.050587 | 0.153355 | 0.350837 | 2.020705 | 7.206859 | 8.925306 |
| discharge | 0.001791 | 0.007722 | 0.024235 | 0.097816 | 0.435813 | 1.325813 | 4.444314 |

**Wilcoxon / Kruskal-Wallis Tests (Rank Sums)**

| Level | Count | Score Sum | Score Mean | (Mean-Mean0)/Std0 |
|---|---|---|---|---|
| death | 12 | 370 | 30.8333 | 2.189 |
| discharge | 34 | 711 | 20.9118 | -2.189 |

**2-Sample Test, Normal Approximation**

| S | Z | Prob>|Z| |
|---|---|---|
| 370 | 2.18887 | 0.0286 |

**1-way Test, ChiSquare Approximation**

| ChiSquare | DF | Prob>ChiSq |
|---|---|---|
| 4.8461 | 1 | 0.0277 |

## Appendix 17

**Contingency Table**
Risk of Mortality By OUTCOME

| Count<br>Total %<br>Col %<br>Row % | death | discharge | |
|---|---|---|---|
| **High** | 5<br>8.06<br>29.41<br>83.33 | 1<br>1.61<br>2.22<br>16.67 | 6<br>9.68 |
| **Intermediate** | 12<br>19.35<br>70.59<br>26.67 | 33<br>53.23<br>73.33<br>73.33 | 45<br>72.58 |
| **Low** | 0<br>0.00<br>0.00 | 11<br>17.74<br>24.44 | 11<br>17.74 |

(continued)

| Contingency Table | | | |
|---|---|---|---|
| Risk of Mortality By OUTCOME | | | |
| | 0.00 | 100.00 | |
| | 17 | 45 | 62 |
| | 27.42 | 72.58 | |

**Tests**

| Source | DF | -LogLike | RSquare (U) |
|---|---|---|---|
| Model | 2 | 7.618343 | 0.2092 |
| Error | 59 | 28.799550 | |
| C. Total | 61 | 36.417893 | |
| N | 62 | | |

| Test | ChiSquare | Prob>ChiSq |
|---|---|---|
| Likelihood Ratio | 15.237 | 0.0005 |
| Pearson | 13.594 | 0.0011 |

**Appendix 18**

**Tests Between Groups**

| Test | ChiSquare | DF | Prob>ChiSq |
|---|---|---|---|
| Log-Rank | 23.2784 | 2 | <.0001 |
| Wilcoxon | 23.3860 | 2 | <.0001 |

**Appendix 19**

| Contingency Table | | | |
|---|---|---|---|
| Mortality Risk By OUTCOME | | | |
| Count<br>Total %<br>Col %<br>Row % | death | discharge | |
| **0** | 0<br>0.00<br>0.00<br>0.00 | 7<br>22.58<br>31.82<br>100.00 | 7<br>22.58 |
| **1** | 1<br>3.23<br>11.11<br>7.14 | 13<br>41.94<br>59.09<br>92.86 | 14<br>45.16 |
| **2** | 5<br>16.13<br>55.56 | 2<br>6.45<br>9.09 | 7<br>22.58 |

(continued)

| Contingency Table | | | |
|---|---|---|---|
| Mortality Risk By OUTCOME | | | |
| | 71.43 | 28.57 | |
| **3** | | 3 0 3 | |
| | 9.68 | 0.00 | 9.68 |
| | 33.33 | 0.00 | |
| | 100.00 | 0.00 | |
| | 9 | 22 | 31 |
| | 29.03 | 70.97 | |

**Tests**

| Source | DF | -LogLike | RSquare (U) |
|---|---|---|---|
| Model | 3 | 10.885300 | 0.5829 |
| Error | 27 | 7.790348 | |
| C. Total | 30 | 18.675648 | |
| N | 31 | | |

| Test | ChiSquare | Prob>ChiSq |
|---|---|---|
| Likelihood Ratio | 21.771 | <.0001 |
| Pearson | 19.560 | 0.0002 |

## Appendix 20

Mortality Risk

**Tests Between Groups**

| Test | ChiSquare | DF | Prob>ChiSq |
|---|---|---|---|
| Log-Rank | 25.8169 | 3 | <.0001 |
| Wilcoxon | 23.3328 | 3 | <.0001 |

## References

1. American College of Chest Physicians/Society of Critical Care Medicine Consensus Conference: definitions for sepsis and organ failure and guidelines for the use of innovative therapies in sepsis. Crit Care Med, 1992. 20(6): p. 864-74.

2. Angus, D.C., et al., Epidemiology of severe sepsis in the United States: analysis of incidence, outcome, and associated costs of care. Crit Care Med, 2001. 29(7): p. 1303-10.

3. Cohen, J., The immunopathogenesis of sepsis. Nature, 2002. 420(6917): p. 885-91.

4. Swain, S.L., et al., IL-4 directs the development of Th2-like helper effectors. J Immunol, 1990. 145(11): p. 3796-806.

5. Diehl, S. and M. Rincon, The two faces of IL-6 on Th1/Th2 differentiation. Mol Immunol, 2002. 39(9): p. 531-6.

6. Boehm, U., et al., Cellular responses to interferon-gamma. Annu Rev Immunol, 1997. 15: p. 749-95.

7. Murphy, K.M., et al., Signaling and transcription in T helper development. Annu Rev Immunol, 2000. 18: p. 451-94.

8. Deng, J.C., et al., STAT4 is a critical mediator of early innate immune responses against pulmonary Klebsiella infection. J Immunol, 2004. 173(6): p. 4075-83.

9. Ono, S., et al., Severe sepsis induces deficient interferon-gamma and interleukin-12 production, but interleukin-12 therapy improves survival in peritonitis. Am J Surg, 2001. 182(5): p. 491-7.

10. Fisher, C.J., Jr., et al., Treatment of septic shock with the tumor necrosis factor receptor:Fc fusion protein. The Soluble TNF Receptor Sepsis Study Group. N Engl J Med, 1996. 334(26): p. 1697-702.

11. Pachot, A., et al., Longitudinal study of cytokine and immune transcription factor mRNA expression in septic shock. Clin Immunol, 2005. 114(1): p. 61-9.

12. Pachot, A., et al., Messenger RNA expression of major histocompatibility complex class II genes in whole blood from septic shock patients. Crit Care Med, 2005. 33(1): p. 31-8; discussion 236-7.

13. Leon, L.R., A.A. White, and M.J. Kluger, Role of IL-6 and TNF in thermoregulation and survival during sepsis in mice. Am J Physiol. 1998. p. R269-77.

14. Sutherland, A.M., et al., The association of interleukin 6 haplotype clades with mortality in critically ill adults. Arch Intern Med. 2005. p. 75-82.

15. Nagabhushanam, V., et al., Innate inhibition of adaptive immunity: Mycobacterium tuberculosis-induced IL-6 inhibits macrophage responses to IFN-gamma. J Immunol. 2003. p. 4750-7.

16. Wasserman, D., et al., Interferon-gamma in the prevention of severe burn-related infections: a European phase III multicenter trial. The Severe Bums Study Group. Crit Care Med, 1998. 26(3): p. 434-9.

17. Polk, H.C., Jr., et al., A randomized prospective clinical trial to determine the efficacy of interferon-gamma in severely injured patients. Am J Surg. 1992. p. 191-6.

18. Docke, W.D., et al., Monocyte deactivation in septic patients: restoration by IFN-gamma treatment. Nat Med, 1997. 3(6): p. 678-81.

19. Nakos, G., et al., Immunoparalysis in patients with severe trauma and the effect of inhaled interferon-gamma. Crit Care Med, 2002. 30(7): p. 1488-94.

**Claims**

1. A method for predicting a human patients response to an infection, the method comprising determining an IL-23 mRNA value in a sample from the patient, and correlating the value with a likelihood that the patient will develop sepsis in response to infection,
   wherein a low IL-23 mRNA value correlates with a likelihood of the patient developing sepsis in response to infection, and high IL-23 mRNA value correlates with a likelihood that the patient will not develop sepsis in response to infection.

2. A method as claimed in Claim 1 in which the sample is peripheral blood mononuclear cells, wherein a low IL-23 mRNA value correlates with less than 1824 copies of IL-23 mRNA per 10 million copies of $\beta$-actin, and a high IL-23 mRNA value correlates with 1824 or more copies of IL-23 per 10 million copies of beta-actin.

3. A method as claimed in Claim 1 and further predicting the outcome of sepsis in a patient, the method comprising the steps of determining a ratio of IL-27:IL-23 mRNA values in a sample from the patient, wherein a ratio greater than 1.45 correlates with death and a ratio less than 1.45 correlates with survival.

4. A method as claimed in Claim 3 which further includes determining a ratio of IL-10:IFN-$\gamma$ mRNA values in a sample from the patient, wherein a ratio greater than 2.85 correlates with death and a ratio less than 2.85 correlates with survival.

**5.** A method as claimed in Claim 4 comprising a scoring system in which a patient is assigned a score of 1 or 0 depending on whether the ratio of IL-27:IL-23 mRNA values is above or below 1.45, respectively, and a score of 1 or 0 depending on whether the ratio of IL-10:IFN- γ mRNA values is above or below 2.85, respectively, wherein a sum of 2 correlates with high risk of mortality due to sepsis, a sum of 0 correlates with a low risk of mortality due to sepsis, and 1 correlates with an intermediate risk of death due to sepsis.

**6.** A method for stratifying a patient into a sepsis/non-sepsis group, the method comprising the steps of determining a ratio of IL-27:IL-23 mRNA values in a sample from the patient, wherein a ratio greater than 1.45 correlates with sepsis group, and a ratio less than 1.45 correlates with non-sepsis group.

**7.** A method as claimed in any of Claims 2 to 6, in which the IL-27, IL-23, IL-10, and IFN- γ mRNA values are represented by IL-27, IL-23, IL-10 and IFN-γ mRNA copy numbers, respectively, and in which the copy numbers are normalised against a housekeeping gene.

**8.** A method as claimed in Claim 7 in which the housekeeping gene is β-Actin.

**9.** A method as claimed in any preceding Claim, wherein the sample is mononuclear cells from a peripheral blood sample, or white cells isolated in the Buffy Coat layer of a peripheral blood sample.

**10.** A method as claimed in any preceding Claim wherein cDNA for IL-27, IL-23, IL-10, and IFN- γ is amplified and quantified as a surrogate for IL-27, IL-23, IL-10, and IFN-γ mRNA copy number, respectively.

**11.** A method as claimed in any one of the preceding claims wherein the IL-27, IL-23, IL-10, and IFN-γ mRNA copy number is measured in absolute terms by reference to a calibration curve constructed from a standard sample of DNA and normalised to a house keeping gene.

**Patentansprüche**

**1.** Ein Verfahren um die Reaktion zu einer Infektion eines menschlichen Patienten zu prognostizieren, die Methode umfasst einen IL-23 mRNA Wert von einer Probe des Patienten zu bestimmen und die Wahrscheinlichkeit zu bestimmen, daß der Patient als Folge der Infektion eine Sepsis erleidet, worin ein niedriger IL-23 mRNA Wert mit der Wahrscheinlichkeit einer Sepsis als Folge der Infektion korreliert und ein hoher IL-23 mRNA Wert mit der Wahrscheinlichkeit korreliert, daß der Patient keine Sepsis als Folge der Infektion erleiden wird.

**2.** Ein Verfahren wie in Behauptung 1 beschrieben, wo die Blutprobe aus Mononuklarzellen von Peripherblut besteht, wobei ein niedriger IL-23 mRNA Wert korreliert mit weniger als 1824 Exemplaren von IL-23 mRNA per 10 Millionen Exemplaren von β-Aktin, und ein hoher IL-23 mRNA Wert korreliert mit 1824 oder mehr Exemplaren von IL-23 per 10 Millionen Exemplaren von Beta-Aktin.

**3.** Ein Verfahren wie in Behauptung 1 beschreiben und ferner die Sepsisprognose bei einem Patienten; wobei das Verfahren die Schritte zur Bestimmung des Verhältnisses von IL-27: IL-23 mRNA Werten von einer Blutprobe des Patienten beinhaltet, wobei ein Verhältnis von mehr als 1,45 mit dem Tod korreliert und einem Verhältnis von weniger als 1,45 mit Überleben korreliert.

**4.** Ein Verfahren wie in Behauptung 3 beschrieben welches außerdem das Verhältnis von IL-10:IFN-γ mRNA Werten von einer Blutprobe des Patienten bestimmt, wobei ein Verhältnis von mehr als 2,85 mit dem Tod korreliert und ein Verhältnis von weniger als 2,85 mit Überleben korreliert.

**5.** Ein Verfahren wie in Behauptung 4 beschrieben, mit einem Bewertungsverfahren, indem einem Patienten eine Punktezahl von 1 oder 0 zugewiesen wird, je nachdem ob das Verhältnis von IL-27: IL-23 mRNA-Werten über oder unterhalb 1.45 liegt, und eine Punktezahl von 1 oder 0, je nachdem ob das Verhältnis von IL-10: IFN-γ mRNA Werte über oder unter 2,85 liegt, wobei eine Summe von 2 mit einem hohen Sterblichkeitsrisiko aufgrund von Sepsis korreliert, eine Summe von 0 mit einem geringen Sterblichkeitsrisiko aufgrund von Sepsis korreliert und 1 mit einem mittleren Sterblichkeitsrisiko durch Sepsis korreliert.

**6.** Ein Verfahren zur Stratifizierung von Patienten in eine Sepsis / Nicht-Sepsis-Gruppe, wobei das Verfahren die Schritte zur Bestimmung des Verhältnisses von IL-27: IL-23 mRNA Werten von einer Blutprobe des Patienten beinhaltet, wobei ein Verhältnis von mehr als 1,45 mit der Sepsisgruppe korreliert, und ein Verhältnis weniger als 1,45 mit der Nicht-Sepsis-Gruppe korreliert.

**7.** Ein Verfahren wie in den Behauptungen 2 bis 6 beschrieben, in denen die IL-27, IL-23, IL-10 und IFN-γ mRNA Werte vertreten sind durch IL-27, IL-23, IL-10 and IFN-γ mRNA Kopiezahlen, und wo die Kopiezahlen gegen ein Haushaltsgen normiert sind.

**8.** Ein Verfahren wie in Behauptung 7 beschrieben wo das Haushaltsgen β-Aktin ist.

**9.** Ein Verfahren wie in allen vorherigen Behauptungen beschrieben, wo die Blutprobe aus Mononuklarzellen von Peripherblut besteht, oder aus isolierten weissen Blutzellen von der Buffy-Coat-Schicht einer Peripherblutprobe.

**10.** Ein Verfahren wie in allen vorherigen Behauptungen beschrieben worin cDNA von IL-27, IL-23, IL-10 und IFN-γ verstärkt und quantifiziert ist als Surrogat für IL-27, IL-23, IL-10 und IFN-γ mRNA Kopiezahlen.

**11.** Ein Verfahren wie in allen vorherigen Behauptungen beschrieben worin die IL-27, IL-23, IL-10 und IFN-γ mRNA Kopiezahlen aus einer DNA-Standardprobe in absoluten Zahlen anhand einer Eichkurve gemessen werden und zu einem Haushaltsgen normiert sind.

**Revendications**

**1.** Méthode de prédiction de la réponse d'un patient humain à une infection, cette méthode consistant à déterminer la valeur de l'ARNm de l'IL-23 dans un échantillon prélevé chez le patient et à établir une corrélation entre cette valeur et la probabilité d'un développement de sepsie chez ce patient en réponse à cette infection, méthode dans laquelle une faible valeur de l'ARNm de l'IL-23 est corrélée à la probabilité du développement d'une sepsie chez le patient en réponse à l'infection et où une valeur élevée de l'ARNm de l'IL-23 est corrélée à la probabilité du non-développement de sepsie chez le sujet en réponse à l'infection.

**2.** Méthode, telle que revendiquée en revendication 1, consistant à prélever des cellules mononuclées sanguines, dans laquelle une faible valeur de l'ARNm de l'IL-23 est corrélée à moins de 1 824 copies d'ARNim de l'IL-23, par 10 millions de copies d'actine bêta et où une valeur élevée de l'ARNim de l'IL-23 est corrélée à 1 824 copies minimum de l'IL-23 par 10 millions de copies d'actine bêta.

**3.** Méthode, telle que revendiquée en revendication 1, consistant par ailleurs à prédire le résultat d'une sepsie chez un patient, cette méthode comprenant les étapes de détermination du ratio des valeurs de l'ARNm de l'IL-27:IL-23 dans un échantillon prélevé chez le patient, où un ratio supérieur à 1,45 est corrélé au décès et où un ratio inférieur à 1,45 est corrélé à la survie.

**4.** Méthode, telle que revendiquée en revendication 3, consistant d'autre part à déterminer le ratio des valeurs de l'ARNm de l'IL-10 IFN-γ dans un échantillon prélevé chez le patient, où un ratio supérieur à 2,85 est corrélé au décès et où un ratio inférieur à 2,85 est corrélé à la survie.

**5.** Méthode, telle que revendiquée en revendication 4, comprenant un barème selon lequel on affecte au patient un score de 1 ou de 0, selon que le ratio des valeurs de l'ARNm de l'IL-27:IL-23 est supérieur ou inférieur à 1,45 respectivement et un score de 1 ou de 0 selon que le ratio des valeurs de l'ARNm de l'IL-10:IFN-γ est supérieur ou inférieur à 2,85 respectivement, méthode dans laquelle une somme de 2 est corrélée à un haut risque de mortalité due à la sepsie, une somme de 0 est corrélée à un faible risque de mortalité due à la sepsie et une somme de 1 est corrélée à un risque immédiat de décès dû à la sepsie.

**6.** Méthode de stratification des patients entre un groupe de sepsie/non-sepsie, cette méthode comprenant les étapes de détermination d'un ratio des valeurs de l'ARNm de l'IL-27:IL-23 dans un échantillon prélevé chez le patient, où un ratio supérieur à 1,45 est corrélé au groupe de sepsie et un ratio inférieur à 1,45 et corrélé au groupe de non sepsie.

**7.** Méthode, telle que revendiquée en revendications 2 à 6, où les valeurs de l'ARNm de l'IL-27, de l'IL-23, de l'IL-10 et de l'IFN-γ sont représentées par le nombre de copies de l'ARNm de l'IL27, de l'IL-23, de l'IL-10 et de l'IFN-γ respectivement et où le nombre de copies est normalisé par rapport à un gène d'entretien.

8. Méthode, telle que revendiquée en revendication 7, où le gène d'entretien est l'actine bêta.

9. Méthode, telle que revendiquée dans la revendication précédente, où l'échantillon consiste de cellules mononuclées sanguines ou de globules blancs isolés dans la couche « buffy coat » du sang périphérique prélevé.

10. Méthode, telle que revendiquée dans la revendication précédente, où l'ADNc de l'IL-27, de l'IL-23, de l'IL-10 et de l'IFN-$\gamma$ est amplifié et quantifié comme substitut du nombre de copies de l'ARNm de l'IL-27, de l'IL-23, de l'IL-10 et de l'IFN-$\gamma$ respectivement.

11. Méthode, telle que revendiquée dans une revendication précédente quelconque, où le nombre de copies de l'ARNm de l'IL-27, de l'IL-23, de l'IL-10 et de l'IFN-$\gamma$ est mesuré en termes absolus par rapport à une courbe d'étalonnage construite à partir d'un échantillon standard d'ADN et normalisée par rapport à un gène d'entretien.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Figure 21

MCS A

       45
loxP    •

TTA TCA GTC GAC GGT ACC GGA CAT ATG CCC GGG AA TTCG GCC ATT ACG GCC TGC AGG ATC C Stuffer fragment.
     Sal I          Nde I  Sma I  EcoR I    Sfi I      Pst I   BamH I

MCS B  292

...Stuffer fragment GG ATC CGG CCG CCT CGG CCC TCG AGA AGC TTT CTA GAC CAT TCG TTT GGC GCG CGG GCC CAG TAG GTA AGT G
      BamH I      Sfi I        Xho I    Hind III   Xba I                      Bsp120 I
                                                                              Apa I

                                                                    STOPs

Figure 22

Figure 23a

Figure 23b

**EP 1 951 899 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004108957 A

**Non-patent literature cited in the description**

- *Crit Care Med,* 1992, vol. 20 (6), 864-74
- **Angus, D.C. et al.** Epidemiology of severe sepsis in the United States: analysis of incidence, outcome, and associated costs of care. *Crit Care Med,* 2001, vol. 29 (7), 1303-10
- **Cohen, J.** The immunopathogenesis of sepsis. *Nature,* 2002, vol. 420 (6917), 885-91
- **Swain, S.L. et al.** IL-4 directs the development of Th2-like helper effectors. *J Immunol,* 1990, vol. 145 (11), 3796-806
- **Diehl, S. ; M. Rincon.** The two faces of IL-6 on Th1/Th2 differentiation. *Mol Immunol,* 2002, vol. 39 (9), 531-6
- **Boehm, U. et al.** Cellular responses to interferon-gamma. *Annu Rev Immunol,* 1997, vol. 15, 749-95
- **Murphy, K.M. et al.** Signaling and transcription in T helper development. *Annu Rev Immunol,* 2000, vol. 18, 451-94
- **Deng, J.C. et al.** STAT4 is a critical mediator of early innate immune responses against pulmonary Klebsiella infection. *J Immunol,* 2004, vol. 173 (6), 4075-83
- **Ono, S. et al.** Severe sepsis induces deficient interferon-gamma and interleukin-12 production, but interleukin-12 therapy improves survival in peritonitis. *Am J Surg,* 2001, vol. 182 (5), 491-7
- **Fisher, C.J., Jr. et al.** Treatment of septic shock with the tumor necrosis factor receptor:Fc fusion protein. The Soluble TNF Receptor Sepsis Study Group. *N Engl J Med,* 1996, vol. 334 (26), 1697-702

- **Pachot, A. et al.** Longitudinal study of cytokine and immune transcription factor mRNA expression in septic shock. *Clin Immunol,* 2005, vol. 114 (1), 61-9
- **Pachot, A. et al.** Messenger RNA expression of major histocompatibility complex class II genes in whole blood from septic shock patients. *Crit Care Med,* 2005, vol. 33 (1), 31-8
- **Leon, L.R. ; A.A. White ; M.J. Kluger.** Role of IL-6 and TNF in thermoregulation and survival during sepsis in mice. *Am J Physiol.,* 1998, R269-77
- **Sutherland, A.M. et al.** The association of interleukin 6 haplotype clades with mortality in critically ill adults. *Arch Intern Med.,* 2005, 75-82
- **Nagabhushanam, V. et al.** Innate inhibition of adaptive immunity: Mycobacterium tuberculosis-induced IL-6 inhibits macrophage responses to IFN-gamma. *J Immunol.,* 2003, 4750-7
- **Wasserman, D. et al.** Interferon-gamma in the prevention of severe burn-related infections: a European phase III multicenter trial. The Severe Bums Study Group. *Crit Care Med,* 1998, vol. 26 (3), 434-9
- **Polk, H.C., Jr. et al.** A randomized prospective clinical trial to determine the efficacy of interferon-gamma in severely injured patients. *Am J Surg.,* 1992, 191-6
- **Docke, W.D. et al.** Monocyte deactivation in septic patients: restoration by IFN-gamma treatment. *Nat Med,* 1997, vol. 3 (6), 678-81
- **Nakos, G. et al.** Immunoparalysis in patients with severe trauma and the effect of inhaled interferon-gamma. *Crit Care Med,* 2002, vol. 30 (7), 1488-94